# EUROPEAN PATENT APPLICATION

(11) **EP 4 382 530 A1**
(43) Date of publication of application: **12.06.2024**
(21) Application number: 22852225.6
(22) Date of filing: 03.08.2022
(51) Int. Cl.: C07J 43/00, A61K 31/495, A61P 35/00

(54) **SHP2 INHIBITOR, PHARMACEUTICAL COMPOSITION COMPRISING SAME, AND APPLICATION THEREOF**

(30) Priority: 04.08.2021 CN 202110891517
(71) Applicant: Beijing Tide Pharmaceutical Co., Ltd., Beijing 100176 (CN)
(72) Inventor: LI, Wenming, Beijing 100176 (CN); LI, Xiaobo, Beijing 100176 (CN); PIAO, Mingnan, Beijing 100176 (CN); LU, Peng, Beijing 100176 (CN); WANG, Ning, Beijing 100176 (CN); YU, Guokun, Beijing 100176 (CN); LIU, Junrong, Beijing 100176 (CN); ZHANG, Yu, Beijing 100176 (CN); YU, Tiantian, Beijing 100176 (CN)
(74) Representative: Ipside
(86) International application number: PCT/CN2022/109904
(87) International publication number: WO 2023/011513

(57) **Abstract**

The present invention provides compounds, or pharmaceutically acceptable salts, esters, optical isomers, stereoisomers, polymorphs, solvates, N-oxides, isotopically labeled compounds, metabolites, chelates, complexes, clathrates, or prodrugs thereof, and pharmaceutical compositions containing the compounds of the invention. Also provides the application of the compounds in preparing SHP2 phosphatase related disease medicaments. The present invention also provides a method for treating a SHP2 phosphatase-Related disease.

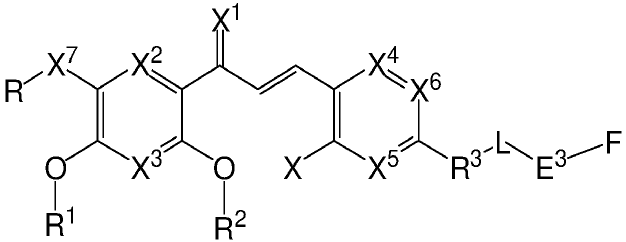

## Description

### Technical Field

. The invention belongs to the field of medicinal chemistry, and particularly relates to an SHP2 inhibitor, a pharmaceutical composition containing the same and medical application thereof.

### Background of the Invention

. SHP2 (The Src homology domain, The Src homology-2domain) is a non-Receptor tyrosine phosphatase encoded by PTPN11 gene. It is ubiquitously expressed in various tissues and cell types and comprises a conserved tyrosine phosphatase domain, two N-terminal SH2 domains, and a C-terminal tail. The two SH2 domains determine the subcellular localization and functional regulation of SHP2. In the inactive state, the N-terminal SH2 domain binds to the PTP domain and inactivates it. When the SH2 domain binds to the receptor or to a specific tyrosine residue on the adaptor protein, the PTP domain is released. For example, stimulation by cytokines and growth factors results in exposure of catalytic sites, resulting in activation of SHP2. SHP2 plays an important role in the regulation of various signaling pathways in cellular biological processes, and is involved in the signaling pathways of various growth factors and interleukins. Within a single signaling pathway, SHP2 may play both an active (signal enhancement) and a passive (signal attenuation) role in the intracellular signaling process. SHP2 is believed to function by dephosphorylating its associated signaling molecule, thereby attenuating local signaling currents. However, the primary role of SHP2 activity in most signaling pathways (e.g., growth factors, interleukins, and extracellular matrix receptors) is to enhance signal transduction. For example, SHP2 is a positive regulator of the ERK/MAPK signaling pathway, which plays a key role in regulating cell proliferation and survival. (SHP2 phosphatase overview see, for example, K. S. Grossman et al, Adv. Cancer Res. 2010, 106, 53-89; and references cited therein).

In the basal state, SHP2 is normally self-inhibited by intramolecular interaction between its N-terminal SH2(N-SH2) domain and its catalytic (PTP) domain, blocking access to the catalytic site. Activation of the protein interacting with the SH2 domain induces a conformational change that reverses this inhibition and allows access of the substrate to the catalytic site. Mutations in the PTPN11 gene at N-SH2 or the PTP domain residues involved in the basic inhibition of SHP2 produce a more activated form of SHP2 protein that causes unregulated or increased SHP2 activity. SHP2 is widely expressed and is involved in multiple cell signaling processes, such as Ras-Erk, PI3K-Akt, Jak-Stat, Met, FGFR, EGFR, insulin receptor and NF-kB pathway, and plays an important role in cell proliferation, differentiation, cell cycle and migration.

Hyperactivation of SHP2 by germline or somatic mutations has been found in Noonan Syndrome, Leopard Syndrome, juvenile myelomonocytic leukemia, myelodysplastic Syndrom, B-Cell acute lymphocytic leukemia, and acute myelogenous leukemia. In addition, activating mutations of PTPN11 are also found in solid tumors, such as lung cancer, colon cancer, melanoma, neuroblastoma, and liver cancer. Therefore, activated SHP2 or up-Regulated SHP2 protein in human tumors or other diseases is a new therapeutic target, and development of SHP2 inhibitors is urgently needed.

### Summary of the Invention

. The inventor of the invention finds that the diaryl acrylketone compound shown in the following Formula (1) has SHP2 inhibitory activity, so that an SHP2 small-molecule inhibitor can be provided, and the activity of targeted degradation of SHP2 is embodied. Specifically, the invention provides a compound shown in Formula 1 or a pharmaceutically acceptable salt, ester, optical isomer, stereoisomer, polymorph, solvate, N-oxide, isotopically labeled compound, metabolite, chelate, complex, clathrate or prodrug thereof,

A compound of Formula 1 or a pharmaceutically acceptable salt, ester, optical isomer, stereoisomer, polymorph, solvate, N-oxide, isotopically labeled compound, metabolite, chelate, complex, clathrate, or prodrug thereof,

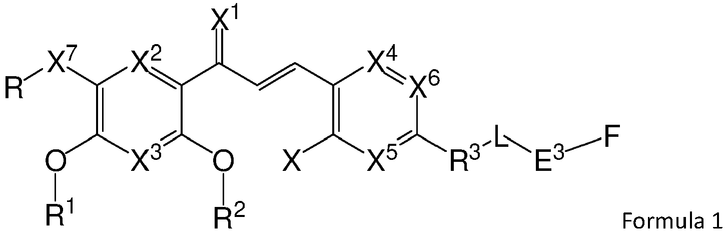

In Formula 1,
X is halogen, C1-6 alkyl, C2-6 alkenyl, C2-6 alkynyl, saturated or partially unsaturated C3-10 cyclic hydrocarbyl, preferably halogen, methyl, ethyl, isopropyl, cyclopropyl, more preferably halogen,
X¹ is NR⁴, O or S, preferably O or S, more preferably O, wherein,
X⁷ is selected from a chemical bond, C1-6 alkylene, C2-6 alkenylene, C2-6 alkynylene, saturated or partially unsaturated C3-10 cyclic hydrocarbylene, -O-, -CO-, -C(=O)O-, -CONH-, -NHCO-, -NHCONH-, -NH-, -S-, sulfinyl, or sulfonyl, preferably C1-6 alkylene, more preferably methylene, ethylene, propylene, or -O-,
X², X³, X⁴, X⁵ and X⁶ are each independently CR⁴ or N, preferably CR⁶,
R¹ is H, C1-6 alkyl, C2-6 alkenyl, C2-6 alkynyl, saturated or partially unsaturated C3-10 cyclic hydrocarbyl, preferably C1-6 alkyl, more preferably one selected from methyl, ethyl, isopropyl and cyclopropyl,
R² is H, C1-6 alkyl, C2-6 alkenyl, C2-6 alkynyl, saturated or partially unsaturated C3-10 cyclic hydrocarbyl, preferably H, C1-6 alkyl, more preferably one selected from H, methyl and ethyl, particularly preferably H,
R³ is CR⁴R⁵, NR⁴, 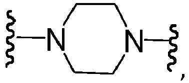 O or S, preferably NR⁴, 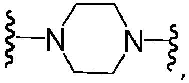 O or S, more preferably NR⁴, 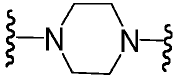 or O,
R⁴ and R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R⁶ is H, halogen, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R is H, halogen, NH₂, OH, SH, amino, substituted or unsubstituted C2-10 aliphatic hydrocarbon group, substituted or unsubstituted saturated or partially unsaturated 3-10 membered heterocyclyl, substituted or unsubstituted C6-10 aryl, or substituted or unsubstituted 5-14 membered heteroaryl group;
R is preferably halogen, OH, SH, haloalkyl, amino, saturated C3-10 cyclic hydrocarbyl, saturated or partially unsaturated 3-10 membered heterocyclyl, C1-6 alkyl substituted amino, 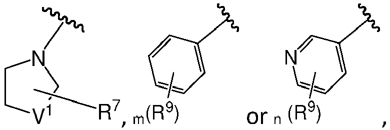 wherein V¹ is one selected from C(R⁸)₂, C(R⁸)₂ -C(R⁸)₂, CR⁸ =CR⁸, C=O, C(=O)C(R⁸)₂, C(R⁸)₂C(=O), C(=O)O, OC(=O), C(=O)NR, N=CR⁸, CR⁸=N, NR⁸-C(R⁸)₂ or C(R⁸)₂-NR⁸;
R⁷ is one selected from H, halogen, cyano, nitro, hydroxylmethyl, hydroxyl, amide, C1-C6 alkyl, C1-C6 alkoxy, C1-C6 alkoxy-C1-C6 alkylene, or 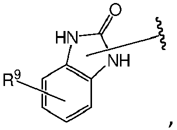
R⁹ are each independently selected from the group consisting of halogen, hydroxyl, oxo, amino, cyano, nitro, -Si(R⁸)₃, C1-6 alkyl, C1-6 alkoxy, saturated or partially unsaturated C3-6 cyclic hydrocarbyl, saturated or partially unsaturated 3-10 membered heterocyclyl, C6-10 aryl, 5-14 membered heteroaryl, C6-12 aralkyl, -C(=O)R⁸, -OC(=O)R⁸, -C(=O)OR⁸,-OR⁸, -SR⁸, -S(=O)R⁸, -S(=O)₂R⁸, -S(=O)₂N(R⁸)₂, -N(R⁸)₂, -C(=O)N(R⁸)₂, -NR⁸-C(=O)R⁸, -NR⁸-C(=O)OR^{B}, -NR⁸-S(=O)₂-R⁸, -NR⁸-C(=O)-N(R⁸)₂, -C1-6 alkylene-N(R⁸)₂, -C1-6 alkylene-OR⁸, -C1-6 alkenylene-OR⁸ and-O-C1-6 alkylene-N(R⁸)₂; m is an integer of 0 to 4, n is an integer of 0 to 3,
R⁸ is H, C1-C6 alkyl, saturated or partially unsaturated C₃₋₆ cyclic hydrocarbyl, saturated or partially unsaturated 3-10 membered heterocyclyl, or C6-10 aryl, the expression of the ring structure with "-" represents the connecting site at any position on the ring structure that can form a bond;
L is a linking group which represents a linear or branched C3-C29 alkylene chain, wherein said linear or branched C3-C29 alkylene chain is optionally interrupted one or more times by one or more divalent groups selected from-O-, -CO-, -C(=O)O-, -CONH-, -NHCO-, -NHCONH-, -NH-, -NR⁸-, -C(R⁸)₂-, -S-, sulfinyl, sulfonyl, sulfinyloxy, sulfonyloxy, -aminosulfonylamino-, alkynylene, alkenylene, cyclic hydrocarbylene, 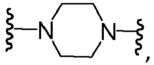 or any combination thereof,
E³ is the following group,

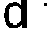

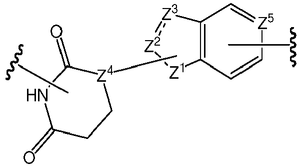

-̅-̅-̅-̅-̅ Represents a single or double bond, preferably a single bond;
Z¹ is O, S, NH, CH₂ or C=O, preferably CH₂ or C=O;
When -̅-̅-̅-̅-̅ is a double bond, Z² is N or CH, and Z³ is N or CH;
When -̅-̅-̅-̅-̅ is a single bond, Z² is O, S, NH, CH₂ or C=O, preferably NH; Z³ is O, S, NH, CH₂ or C=O, preferably CH₂ or C=O;
Z⁴ is N or CH, and Z⁴ is connected with any connectable position of Z¹, Z² and Z³;
Z⁵ is N or CH, preferably N;
E³ is more preferably 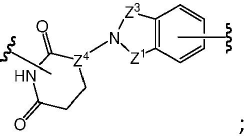
F is H or 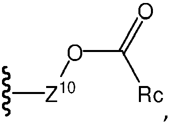
Z¹⁰ is selected from a chemical bond, C1-6 alkylene, C2-6 alkenylene, C2-6 alkynylene, saturated or partially unsaturated C3-10 cyclic hydrocarbylene, preferably a bond, C1-6 alkylene;
Rc is halogen, cyano, nitro, hydroxyl, amido, C1-C6 alkyl, C1-C6 alkoxy, substituted C1-C6 alkyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, preferably C1-C6 alkyl, saturated or partially unsaturated C3-10 cyclic hydrocarbyl, C6-10 aryl, C6-12 aralkyl,

In the above expression, 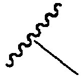 denotes the position of the linkage, and the expression of the ring straucture with "-" represents the connecting site at any position on the ring structure that can form a bond.

The compound designed and synthesized in the invention can effectively inhibit the function of SHP2, can reduce the content of SHP2 in cells, has better activity of resisting solid tumors and blood tumors in vivo and in vitro, and has lower toxicity to normal cells.

The compound of the present application is structurally inventive. This kind of compound can inhibit the phosphatase activity of SHP2, inhibit the signal channel transmission of SHP2 phosphatase dependency, inhibit the activity of proteins such as downstream proteins ERK, STAT3, STATS, c-SRC, JAK2 and the like, and inhibit malignant proliferation and distal metastasis of tumors; meanwhile, can block the downstream signal transmission independent of the important phosphatase of SHP2, inhibit the activity of protooncogenes such as KRAS and NRAS, and stabilize the tumor suppressor gene TP53, thereby achieving the purpose of inhibiting tumor growth, infiltration and metastasis; This kind of compound can also effectively improve the activity of T cells in a tumor microenvironment and prevent tumor cells from escaping immune surveillance to a certain extent. Therefore, the compound of the application can affect the function of SHP2 from two ways of SHP2 phospholipase dependence and phospholipase independence to achieve the aim of resisting tumors, so that the compound can be explained as specifically inhibiting the two functions of SHP2 and can obtain an active compound with high efficiency and low toxicity.

The respective elements of the present invention will be described in more detail below.

### Brief Description of the Drawings

FIG. 1 is a graph showing SHP2 test results of in vitro degradation of the compound of the present invention.
FIG. 2 is a statistical graph of the in vitro degradation SHP2 activity of the compound of the present invention.

### Detailed Description of Embodiments

. The respective elements of the present invention will be described in more detail below.

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

As used herein, the term "alkylene" refers to a saturated divalent hydrocarbyl, preferably refers to a saturated divalent hydrocarbyl having 1, 2, 3, 4, 5 or 6 carbon atoms, e.g., methylene, ethylene, propylene or butylene.

As used herein, the term "alkyl" is defined as a linear or branched saturated aliphatic hydrocarbon. In some embodiments, alkyl has 1-12, e.g., 1-6, carbon atoms. For example, as used herein, the term "C₁₋₆ alkyl" refers to a linear or branched group having 1-6 carbon atoms (such as methyl, ethyl, n-propyl, isopropyl, n-butyl, isobutyl, sec-butyl, tert-butyl, n-pentyl, isopentyl, neopentyl, or n-hexyl), which is optionally substituted with one or more (e.g., 1 to 3) suitable substituents such as halogen (in which case the group may be referred to as "haloalkyl") (e.g., CH₂F, CHF₂, CF₃, CCl₃, C₂F₅, C₂Cl₅, CH₂CF₃, CH₂Cl or-CH₂CH₂CF₃ etc.). The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or tert-butyl).

As used herein, the term "alkenyl" refers to a linear or branched monovalent hydrocarbyl having a double bond and 2-6 carbon atoms ("C₂₋₆ alkenyl"). The alkenyl is e.g., vinyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present invention contains an alkenyl group, the compound may exist as the pure E (entgegen) form, the pure Z (zusammen) form, or any mixture thereof.

As used herein, the term "alkynyl" refers to a monovalent hydrocarbyl containing one or more triple bond, and preferably having 2, 3, 4, 5 or 6 carbon atoms, e.g., ethynyl or propynyl.

As used herein, the term "cycloalkyl" refers to a saturated monocyclic or polycyclic (e.g., bicyclic) hydrocarbon ring (e.g., monocyclic, such as cyclopropyl, cyclobutyl, cyclopentyl, cyclohexyl, cycloheptyl, cyclooctyl, or cyclononyl, or bicyclic, including spiro, fused or bridged cyclic system (such as bicyclo[1.1.1]pentyl, bicyclo[2.2.1]heptyl, bicyclo[3.2.1]octyl or bicyclo[5.2.0] nonyl, or decahydronaphthalene etc.)), which is optionally substituted with one or more (e.g., 1 to 3) suitable substituents. The cycloalkyl has 3 to 15 carbon atoms. For example, the term "C₃₋₆ cycloalkyl" refers to a saturated monocyclic or polycyclic (e.g., bicyclic) hydrocarbon ring having 3 to 6 ring forming carbon atoms (e.g., cyclopropyl, cyclobutyl, cyclopentyl, or cyclohexyl), which is optionally substituted with one or more (e.g., 1 to 3) suitable substituents, e.g., methyl substituted cyclopropyl.

As used herein, the terms "cyclic hydrocarbylene", "cyclic hydrocarbyl" and "hydrocarbon ring" refer to a saturated (i.e., "cyclic hydrocarbylene" and "cycloalkyl") or unsaturated (i.e., having one or more double and/or triple bonds in the ring) monocyclic or polycyclic hydrocarbon ring having e.g., 3-10 (suitably having 3-8, and more suitably having 3-6) ring carbon atoms, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the terms "heterocycly", "heterocyclylene" and "heterocycle" refer to a saturated (i.e., heterocycloalkyl) or partially unsaturated (i.e., having one or more double and/or triple bonds in the ring) cyclic group having e.g. 3-10 (suitably having 3-8, and more suitably having 3-6) ring atoms, wherein at least one ring atom is a heteroatom selected from the group consisting of N, O and S, and the remaining ring atoms are C. For example, "3- to 10-membered heterocyclyl(ene)" of "3- to 10-membered heterocycle" refers to saturated or partially unsaturated heterocyclyl(ene) or heterocycle having 2-9 (e.g., 2, 3, 4, 5, 6, 7, 8 or 9) ring carbon atoms and one or more (e.g., 1, 2, 3, or 4) heteroatoms independently selected from the group consisting of N, O and S. Examples of heterocyclylene, heterocyclyl and heterocycle include, but are not limited to oxiranyl(ene), aziridinyl(ene), azetidinyl(ene), oxetanyl(ene), tetrahydrofuranyl(ene), dioxolinyl(ene), pyrrolidinyl(ene), pyrrolidonyl(ene), imidazolidinyl(ene), pyrazolidinyl(ene), pyrrolinyl(ene), tetrahydropyranyl(ene), piperidinyl(ene), morpholinyl(ene), dithianyl(ene), thiomorpholinyl(ene), piperazinyl(ene) or trithianyl(ene). Said group also encompasses a bicyclic system, including a spiro, fused, or bridged system (e.g., 8-azaspiro[4.5]decane, 3,9-diazaspiro[5.5]undecane, 2-azabicyclo[2.2.2] octane, etc.). Heterocyclylene, heterocyclyl and heterocycle may optionally be substituted with one or more (e.g. 1, 2, 3 or 4) suitable substituents.

As used herein, the terms "aryl(ene)" and "aromatic ring" refer to an all-Carbon monocyclic or fused-Ring polycyclic aromatic group having a conjugated π electron system. For example, as used herein, the terms "C ₆₋₁₀ aryl(ene)" and "C ₆₋₁₀ aromatic ring" refer to an aromatic group containing 6 to 10 carbon atoms, such as phenyl(ene) (benzene ring) or naphthyl(ene) (naphthalene ring). Aryl(ene) or aromatic ring is optionally substituted with one or more (such as 1 to 3) suitable substituents (e.g., halogen, -OH, -CN, -NO ₂, and C₁₋₆ alkyl, etc.).

As used herein, the term "aralkyl" preferably means aryl or heteroaryl substituted alkyl, wherein aryl, heteroaryl and alkyl are as defined herein. Normally, the aryl group may have 6-14 carbon atoms, the heteroaryl group may have 5-14 ring atoms, and the alkyl group may have 1-6 carbon atoms. Exemplary aralkyl group includes, but is not limited to, benzyl, phenylethyl, phenylpropyl, phenylbutyl.

As used herein, the term "halo" or "halogen" are defined to include F, Cl, Br, or I.

As used herein, the term "nitrogen containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may further optionally comprise one or more (e.g., one, two, three or four) ring members selected from the group consisting of N, O, C=O, S, S=O and S(=O) ₂. The nitrogen containing heterocycle is attached to the rest of the molecule through the nitrogen atom and any other ring atom in said nitrogen containing heterocycle. The nitrogen containing heterocycle is optionally benzO-fused, and is preferably attached to the rest of the molecule through the nitrogen atom in said nitrogen containing heterocycle and any carbon atom in the fused benzene ring.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted", the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more from a list of substituents, one or more of the hydrogens on the nitrogen (to the extent there are any) may each be replaced with an independently selected optional substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen, such as ²H, ³H; carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-d₆, or DMSO-d₆.

The term "stereoisomer" refers to isomers with at least one asymmetric center. A compound having one or more (e.g., one, two, three or four) asymmetric centers can give rise to a racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a keto-enol tautomer, phenol-keto tautomer, nitrosO-oxime tautomer, imine-enamine tautomer and the like. It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The chemical bonds of the compound of the present invention may be depicted herein using a solid line (), a solid wedge (), or a dotted wedge (). The use of a solid line to depict bonds to asymmetric carbon atoms is meant to indicate that all possible stereoisomers (e.g., specific enantiomers, racemic mixtures, etc.) at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include cis and trans isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The present invention includes all possible crystalline forms or polymorphs of the compound of the present invention, either as a single polymorph, or as a mixture of more than one polymorphs, in any ratio.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, ester, solvate, N-oxide, metabolite ,Chelate, complex, inclusion compound or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof, including but not limited to salts containing hydrogen bonds or coordination bonds.

A suitable acid addition salt is formed from an acid which forms a pharmaceutically acceptable salt. Specific examples include acetate, adipate, aspartate, benzoate, besylate, bicarbonate/carbonate, bisulfate/sulfate, borate, camphorsulfonate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-Napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

A suitable base addition salt is formed from a base which forms a pharmaceutically acceptable salt. Specific examples include aluminum, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

As used herein, the term "ester" refers to those derived from the compounds of the various Formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

As can be appreciated by a person skilled in the art, not all nitrogen containing heterocycles can form N-oxides since the nitrogen requires an available lone-pair electron for oxidation to the oxide; a person skilled in the art will recognize those nitrogen containing heterocycles which can form N-oxides. A person skilled in the art will also recognize that tertiary amines can form N-oxides. Synthetic methods for the preparation of N-oxides of heterocycles and tertiary amines are well known to a person skilled in the art, and they include the oxidation of heterocycles and tertiary amines with peroxy acids such as peracetic acid and m-Chloroperbenzoic acid (MCPBA), hydrogen peroxide, alkyl hydroperoxides such as tert-butyl hydroperoxide, sodium perborate, and dioxiranes such as dimethyldioxirane. These methods for the preparation of N-oxides have been extensively described and reviewed in literatures, see e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The metabolite of the compound of the present invention, namely a substance formed in vivo upon administration of the compound of the present invention, is also included within the scope of the present invention. Such a product may result e.g., from the oxidation, reduction, hydrolysis, amidation, de-amidation, esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted in vivo into the compound with desired therapeutic activity. Further information on the use of the prodrug may be found in "PrO-drugs as Novel Delivery Systems", Vol. 14, ACS Symposium Series (T. Higuchi and V. Stella). The prodrug in accordance with the invention can, for example, be produced by replacing appropriate functionalities present in the compound of the present invention with certain moieties known to those skilled in the art as "prO-moieties" as described, for example, in "Design of Prodrugs" by H. Bundgaard (Elsevier, 1985).

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in T. W. Greene & P. G. M. Wuts, Protective Groups in Organic Synthesis, John Wiley & Sons, 1991, which is incorporated herein by reference. The protecting groups may be removed at a convenient subsequent stage using methods known from the art.

The term "about" refers to a range within ±10%, preferably within ±5%, and more preferably within ±2% of the specified value.

### Compound

The compound of the invention has a structure shown in a Formula 1 or a pharmaceutically acceptable salt, ester, optical isomer, stereoisomer, polymorph, solvate, N-oxide, isotopically labeled compound, metabolite, chelate, complex, clathrate or prodrug thereof,

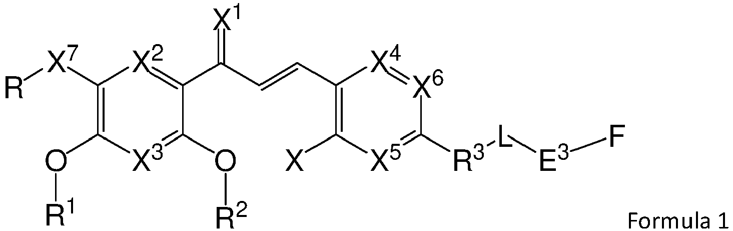

In Formula 1,
X is halogen, C1-6 alkyl, C2-6 alkenyl, C2-6 alkynyl, saturated or partially unsaturated C3-10 cyclic hydrocarbyl, preferably halogen, methyl, ethyl, isopropyl, cyclopropyl, more preferably halogen,
X¹ is NR⁴, O or S, preferably O or S, more preferably O,
X⁷ is one selected from a chemical bond, C1-6 alkylene, C2-6 alkenylene, C2-6 alkynylene, saturated or partially unsaturated C3-10 cyclic hydrocarbylene, -O-, -CO-, -C(=O)O-, -CONH-, -NHCO-, -NHCONH-, -NH-, -S-, sulfinyl, and sulfonyl, preferably C1-6 alkylene, and more preferably methylene, ethylene, propylene, -O-,
X², X³, X⁴, X⁵ and X⁶ are each independently CR⁴ or N, preferably CR⁶,
R¹ is H, C1-6 alkyl, C2-6 alkenyl, C2-6 alkynyl, saturated or partially unsaturated C3-10 cyclic hydrocarbyl, preferably C1-6 alkyl, more preferably one selected from methyl, ethyl, isopropyl and cyclopropyl,
R² is H, C1-6 alkyl, C2-6 alkenyl, C2-6 alkynyl, saturated or partially unsaturated C3-10 cyclic hydrocarbyl, preferably H, C1-6 alkyl, more preferably one selected from H, methyl and ethyl, particularly preferably H,
R³ is CR⁴R⁵, NR⁴, 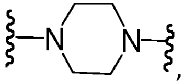 -N(CH₂CH₂)₂N-, O or S, preferably NR⁴, 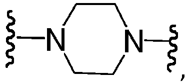 O or S, more preferably NR⁴, 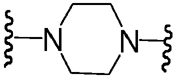 or O,
R⁴ and R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R⁶ is H, halogen, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R is H, halogen, NH₂, OH, SH, substituted or unsubstituted C2-10 aliphatic hydrocarbon group, substituted or unsubstituted saturated or partially unsaturated 3-10 membered heterocyclyl, a substituted or unsubstituted C6-10 aryl, or substituted or unsubstituted 5-14 membered heteroaryl;
R is preferably halogen, OH, SH, haloalkyl, amino, saturated C3-10 cyclic hydrocarbyl, saturated or partially unsaturated 3-10 membered heterocyclyl, C1-6 alkyl substituted amino, 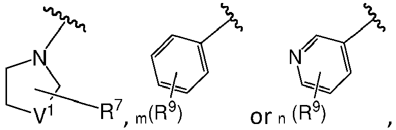 wherein V¹ is one selected from C(R⁸)₂, C(R⁸)₂ -C(R⁸)₂, CR⁸=CR⁸, C=O, C(=O)C(R⁸)₂, C(R⁸)₂C(=O), C(=O)O, OC(=O), C(=O)NR, N=CR⁸, CR⁸=N, NR⁸-C(R⁸)₂ or C(R⁸)₂-NR⁸;
R⁷ is one selected from H, halogen, cyano, nitro, hydroxylmethyl, hydroxyl, amide, C1-C6 alkyl, C1-C6 alkoxy, C1-C6 alkoxy-C1-C6 alkylene, or 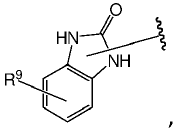
R⁹ are each independently selected from the group consisting of halogen, hydroxyl, oxo, amino, cyano, nitro, -Si(R⁸)₃, C1-6 alkyl, C1-6 alkoxy, saturated or partially unsaturated C3-6 cyclic hydrocarbyl, saturated or partially unsaturated 3-10 membered heterocyclyl, C6-10 aryl, 5-14 membered heteroaryl, C6-12 aralkyl, -C(=O)R⁸, -OC(=O)R⁸, -C(=O)OR⁸,-OR⁸, -SR⁸, -S(=O)R⁸, -S(=O)₂R⁸, -S(=O)₂N(R⁸)₂, -N(R⁸)₂, -C(=O)N(R⁸)₂, -NR⁸-C(=O)R⁸, -NR⁸-C(=O)OR⁸, -NR⁸-S(=O)₂-R⁸, -NR⁸-C(=O)-N(R⁸)₂, -C1-6 alkylene-N(R⁸)₂, -C1-6 alkylene-OR⁸, -C1-6 alkenylene-OR⁸ and-O-C1-6 alkylene-N(R⁸)₂; m is an integer of 0 to 5, n is an integer of 0 to 4,
R⁸ is H, C1-C6 alkyl, saturated or partially unsaturated C₃₋₆ cyclic hydrocarbyl, saturated or partially unsaturated 3-10 membered heterocyclyl, or C6-10 aryl, the expression of the ring structure with "-" represents the connecting site at any position on the ring structure that can form a bond;
L is a linking group which represents a linear or branched C3-C29 alkylene chain, wherein said linear or branched C3-C29 alkylene chain is optionally interrupted one or more times by one or more divalent groups selected from-O-, -CO-, -C(=O)O-, -CONH-, -NHCO-, -NHCONH-, -NH-, -NR⁸-, -C(R⁸)₂-, -S-, sulfinyl, sulfonyl, sulfinyloxy, sulfonyloxy, -aminosulfonylamino-, alkynylene, alkenylene, cyclic hydrocarbylene, 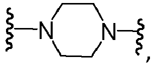 or any combination thereof,
E³ is the following group, 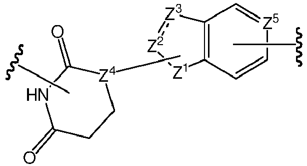
-̅-̅-̅-̅-̅Represents a single or double bond, preferably a single bond;
Z¹ is O, S, NH, CH₂ or C=O, preferably CH₂ or C=O;
When -̅-̅-̅-̅-̅ is a double bond, Z² is N or CH, and Z³ is N or CH;
When -̅-̅-̅-̅-̅ is a single bond, Z² is O, S, NH, CH₂ or C=O, preferably NH; Z³ is O, S, NH, CH₂ or C=O, preferably CH₂ or C=O;
Z⁴ is N or CH, and Z⁴ is connected with any connectable position of Z¹, Z² and Z3.
Z⁵ is N or CH, preferably N;
E³ is more preferably 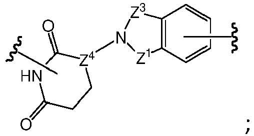
F is H or 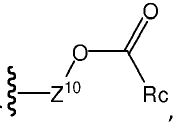
Z¹⁰ is selected from a chemical bond, C1-6 alkylene, C2-6 alkenylene, C2-6 alkynylene, saturated or partially unsaturated C3-10 cyclic hydrocarbylene, preferably a bond, C1-6 alkylene;
Rc is halogen, cyano, nitro, hydroxyl, amido, C1-C6 alkyl, C1-C6 alkoxy, substituted C1-C6 alkyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, preferably C1-C6 alkyl, saturated or partially unsaturated C3-10 cyclic hydrocarbyl, C6-10 aryl, C6-12 aralkyl,

In the above expression, 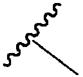 denotes the position of the linkage, and the expression of the ring straucture with "-" represents the connecting site at any position on the ring structure that can form a bond.

In a preferred embodiment, the compound of the present invention has the structure of Formula 2 below, 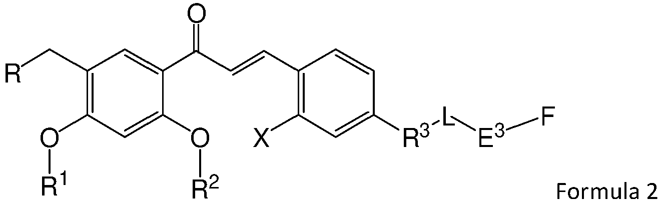
In Formula 2, X is halogen, C1-6 alkyl, saturated or partially unsaturated C3-10 cyclic hydrocarbyl, preferably halogen,
In Formula 2, R¹ is H, C1-6 alkyl, C2-6 alkenyl, C2-6 alkynyl, saturated or partially unsaturated C3-10 cyclic hydrocarbyl, preferably C1-6 alkyl, further preferably ethyl,
In Formula 2, R² is H, C1-6 alkyl, C2-6 alkenyl, C2-6 alkynyl, saturated or partially unsaturated C3-10 cyclic hydrocarbyl, preferably H,
In Formula 2, R³ is NR⁴, 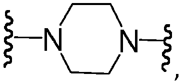 O or S, preferably NR⁴, 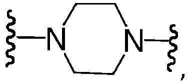 O or S, further preferably NR⁴, 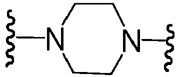 or O, in Formula 2, R⁴ are each independently H, C1-6 alkyl, R⁴ is preferably H,

In Formula 2, R, L, E ³ and F have the same meanings as in Formula 1, and in the above expression, 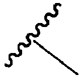 represents a connecting site.

In a preferred embodiment, the compound of the present invention has the structure shown in Formula 3 below, 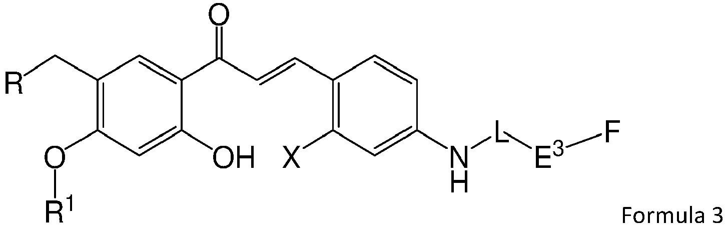
In Formula 3, X is halogen, C1-6 alkyl, saturated or partially unsaturated C3-10 cyclic hydrocarbyl, preferably halogen, R¹ is H, C1-6 alkyl, C2-6 alkenyl, C2-6 alkynyl, saturated or partially unsaturated C3-10 cyclic hydrocarbyl, preferably C1-6 alkyl, further preferably ethyl,
In Formula 3, R, L, E³ and F have the same meanings as in Formula 1, and in the above expression, 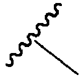 represents a connecting site.
In a preferred embodiment, the compound of the present invention has the structure shown in Formula 4 below, 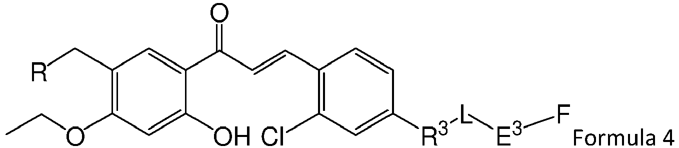
In Formula 4, R³ is CR⁴R⁵, NH, 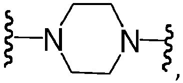 O or S, preferably NR⁴, 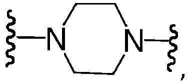 O or S, more preferably NH, 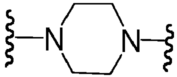 or O, R⁴, R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl,
In Formula 4, R, L, E³ and F have the same meanings as in Formula 1, and in the above expression, 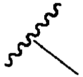 represents a connecting site.
In a preferred embodiment, in the general Formula of the compound of the invention,
E³ is one selected from the following groups,

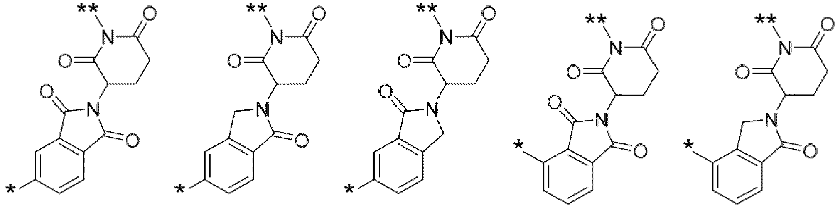

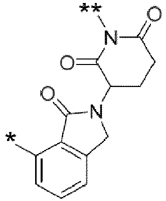

The above groups are linked to L via one of the two positions labeled with *-or **- and the other position is linked to F,
F is H or one of the following groups,

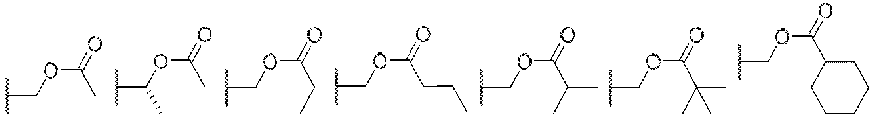

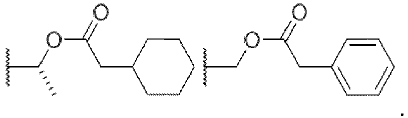

In a preferred embodiment of the present invention,
R is halogen, OH or 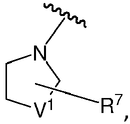 wherein V¹ is one selected from CH₂, CH₂-CH₂, CH=CH, NH-CH₂ or CH₂-NH; R⁷ is one selected from H, C1-C6 alkyl, C1-C6 alkoxy substituted C1-C6 alkylene or 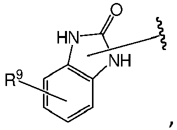 R⁹ is as defined in Formula 1;

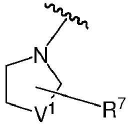

is preferably one selected from 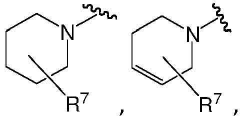 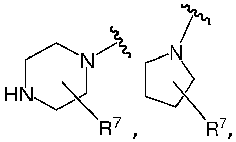 wherein R⁷ is further preferably one selected from H, methyl, ethyl, 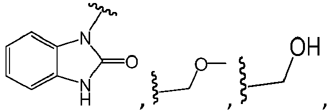 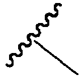 represents a connecting site.

In another preferred embodiment, in the general Formula of the compound of the invention, R is one of the following groups, 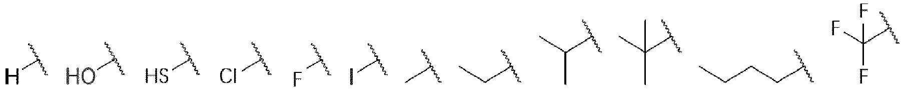 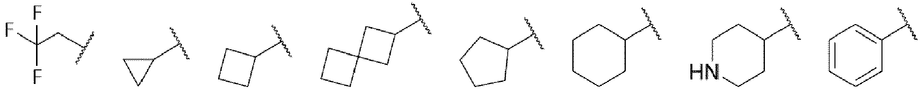 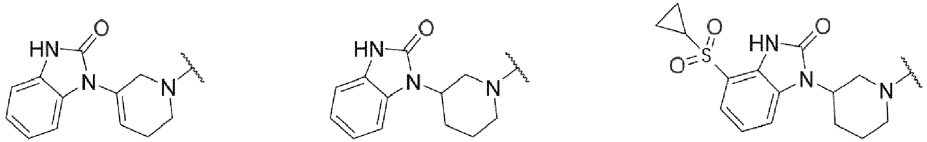 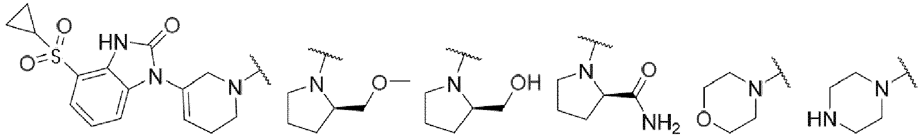 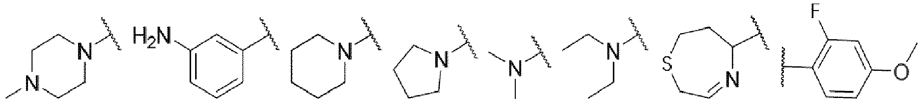 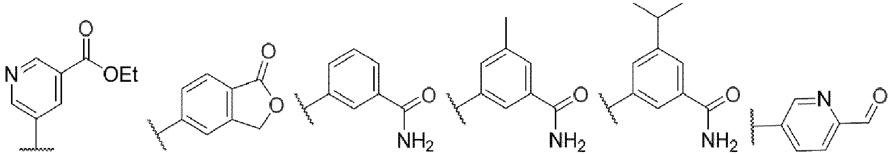 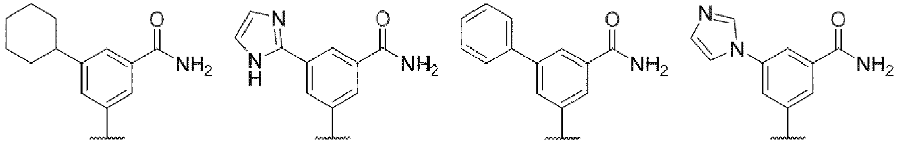 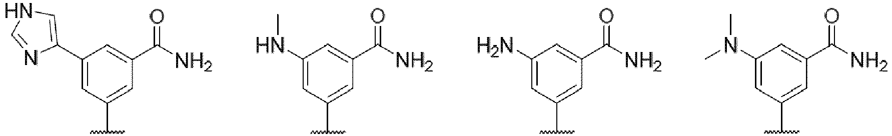 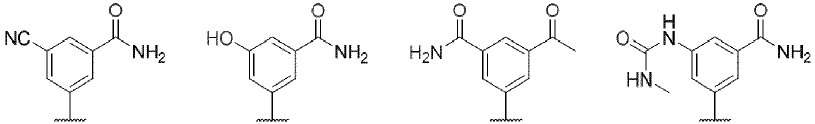 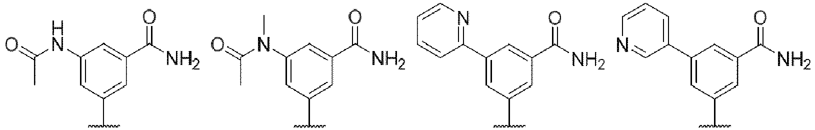 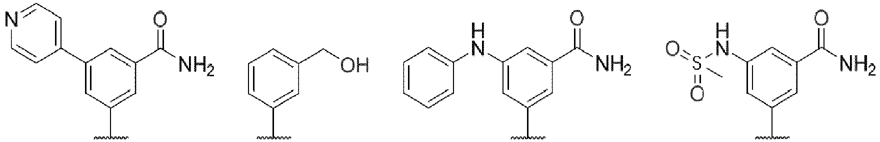 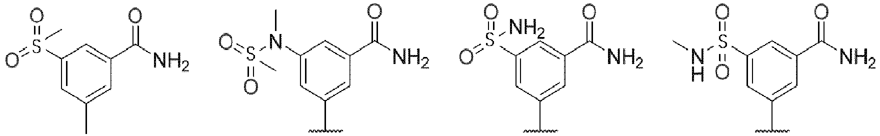 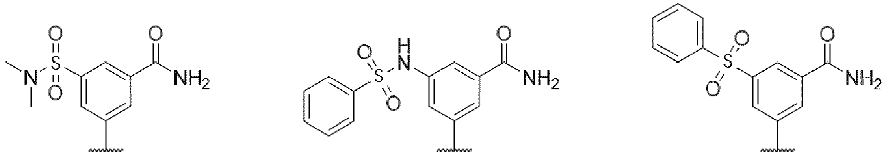 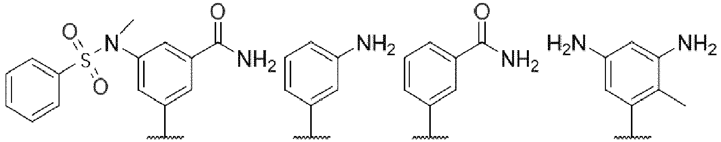 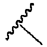 represents a connecting site.

In a preferred embodiment, in the general Formula of the compound of the present invention, L is a divalent group represented by Formula 5,

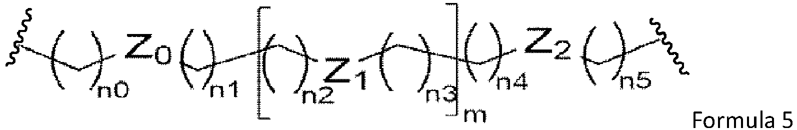

n₀ is 0 or 1,
m is an integer from 1 to 5, preferably 2 or 3; n₁ is an integer from 0 to 3, preferably 1; n₂ is an integer of 0 to 3, preferably 1; n₃ is an integer from 0 to 3, preferably 1; n₄ is an integer of 0 to 3, preferably 1; n₅ is an integer of 0 to 3, preferably 1;
Z₀ is -CH₂-, -NH-, -O-, -S-, 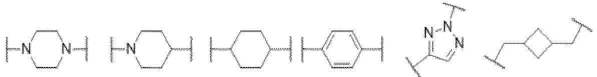 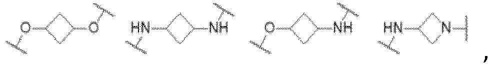 -CO- or -C(=O)O-;
Z₁ is -CH₂-, -NH-, -O-, -S-, 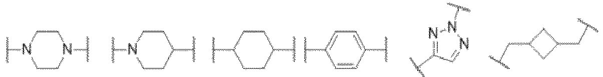 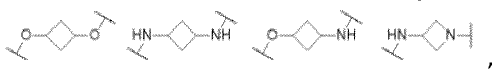 -CO- or -C(=O)O-;
Z₂ is -CH₂-, -NH-, -O-, -S-, 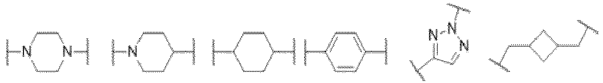 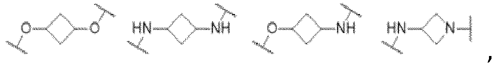 -CO- or -C(=O)O-; 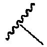
represents a connecting site.

In another preferred embodiment, L is one of the following divalent groups, 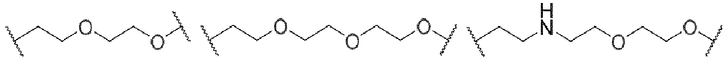 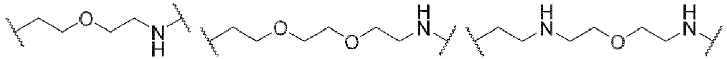 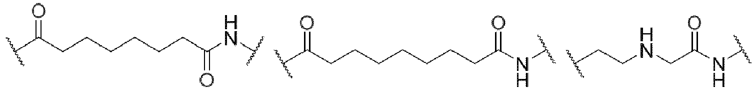 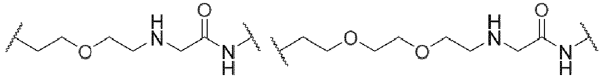 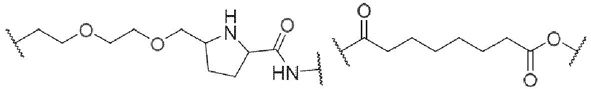 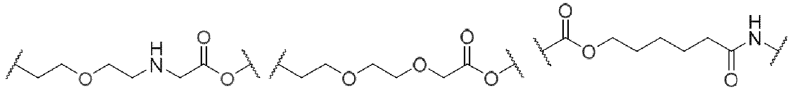 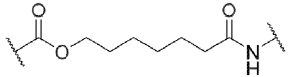 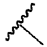 represents a connecting site.

Although the following structures are typical specific structures for describing the compound of the present invention in more detail, the present invention is not limited to the following structures, and the numbers and molecular weights of the corresponding molecules are shown in the table.

| | | |
|---|---|---|
| **TDC.20 01** | 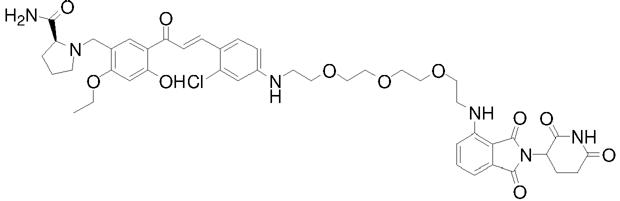 | 875.3 7 |
| **TDC.20 02** | 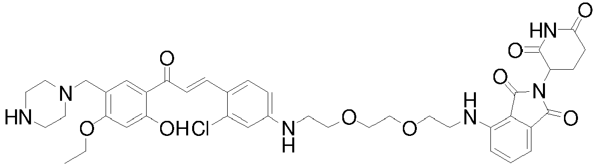 | 803.3 1 |
| **TDC.20 03** | 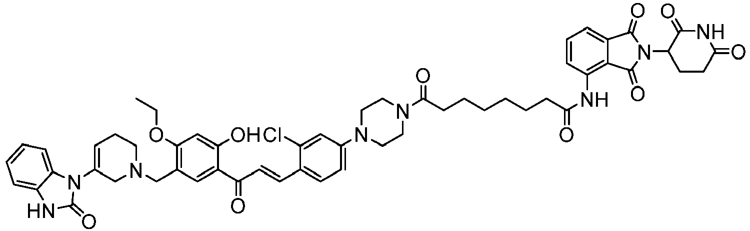 | 1025. 56 |
| **TDC.20 04** | 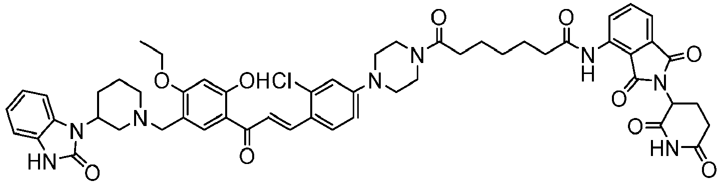 | 1013. 55 |
| **TDC.20 05** | 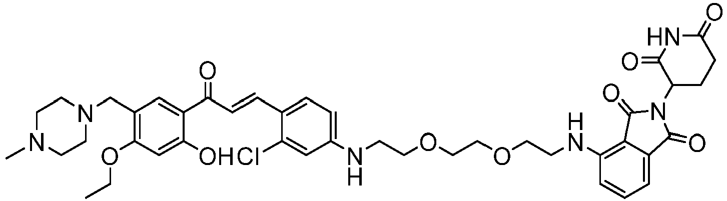 | 817.3 4 |
| **TDC.20 06** | 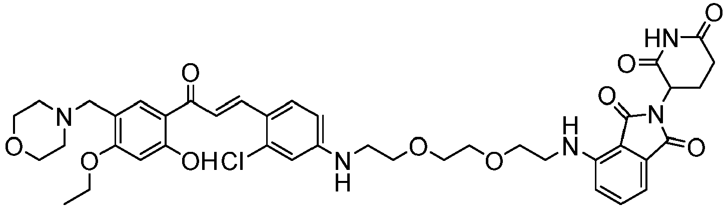 | 804.2 9 |
| **TDC.20 07** | 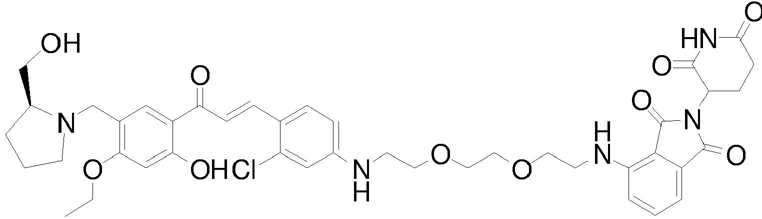 I | 818.3 2 |
| **TDC.20 08** | 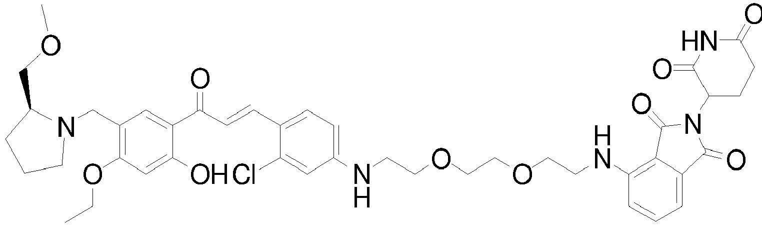 I | 832.3 5 |
| **TDC.20 09** | 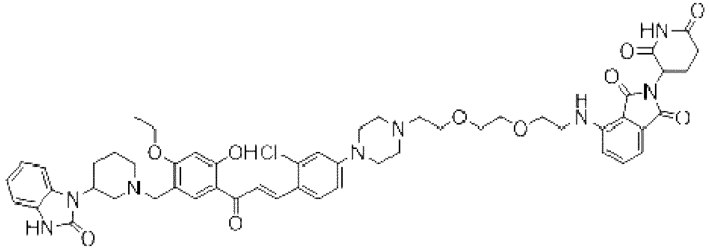 | 1003. 55 |
| **TDC.20 10** | 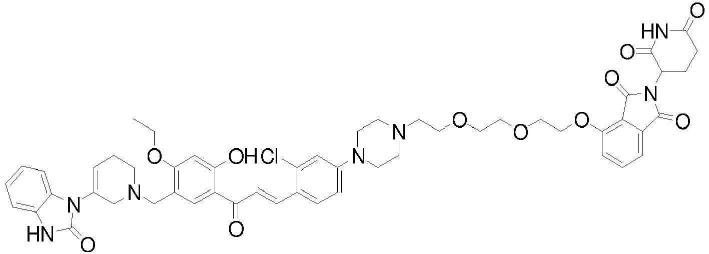 | 1002. 52 |
| **TDC.20 11** | 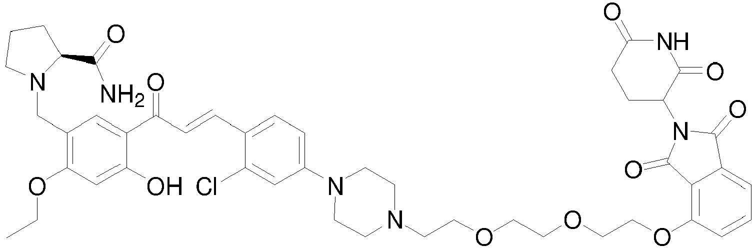 | 901.4 1 |
| **TDC.20 12** | 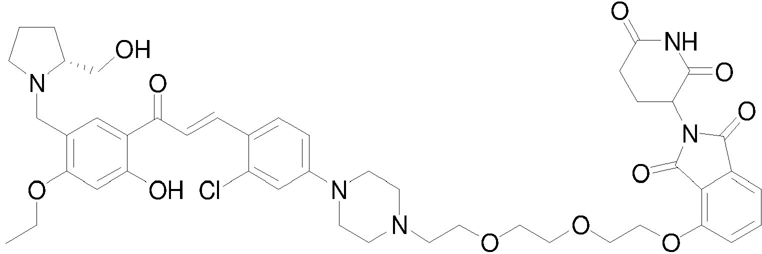 | 888.4 1 |
| **TDC.20 13** | 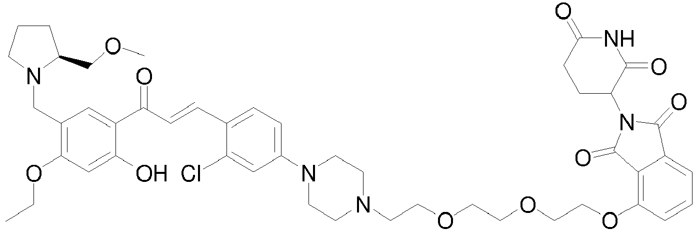 | 902.4 4 |
| **TDC.20 14** | 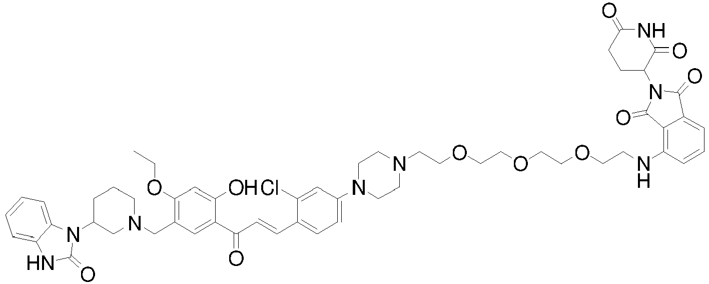 | 1047. 60 |
| **TDC.20 15** | 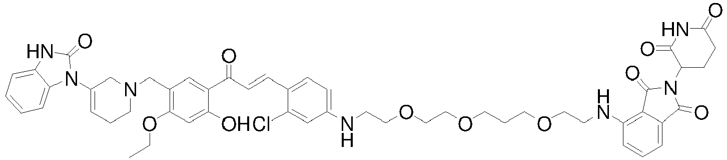 | 990.5 1 |
| **TDC.20 16** | 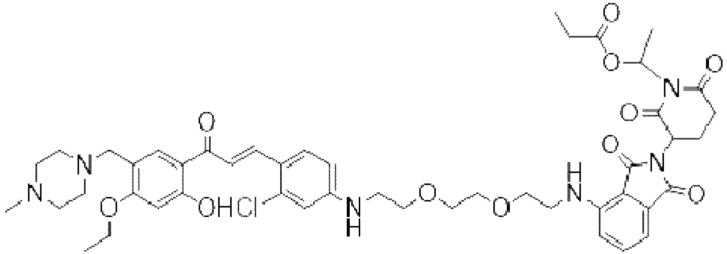 | 917.4 5 |
| **TDC.20 17** | 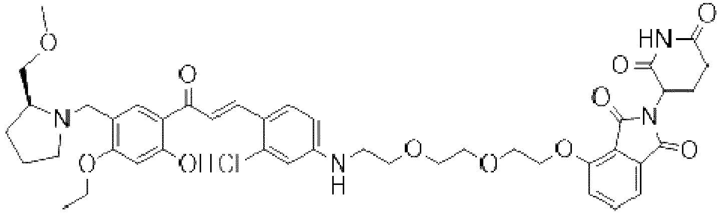 | 876.4 0 |
| **TDC.20 18** | 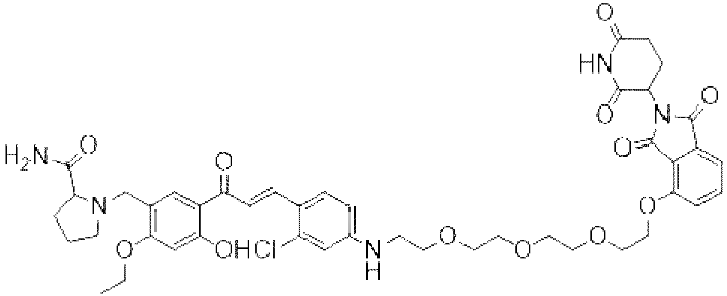 | 876.3 6 |
| **TDC.20 19** | 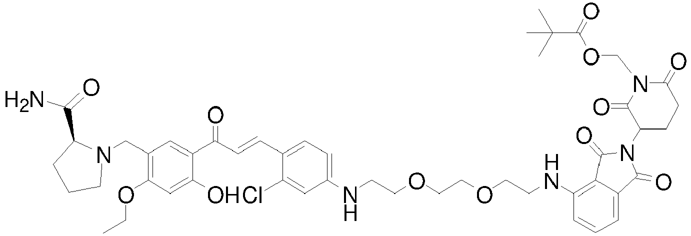 | 945.4 6 |
| **TDC.20 20** | 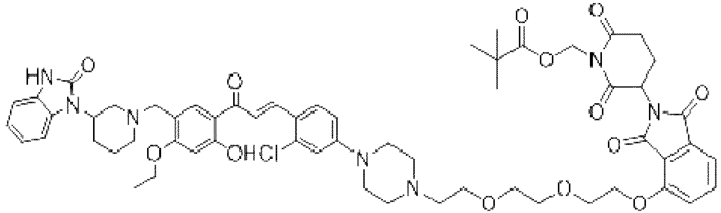 | 1118. 68 |

### General procedure for obtaining compounds of the invention

The compound represented by general Formula 1 of the present invention can be obtained by a known method, for example, by a known organic synthesis method. Exemplary synthetic routes are given below, but may be obtained by other methods known to those skilled in the art.

In the present invention, a method for synthesizing the compounds is briefly described, a representative synthetic route of the compound of the general Formula 1 is as follows, it should be noted that, here, a synthetic route of the compound of the general Formula 1 in which X¹ is O, and X², X³, X⁴, X⁵ are all CH is taken as an example, and the synthesis of the compound in which X¹-X⁵ are other parallel technical solutions can be realized by replacing corresponding raw materials.

Representative technical scheme 1:
[1] Taking I and II as starting materials, in an organic solvent such as dichloromethane, trichloromethane, ethyl acetate or DMF and the like, under alkaline conditions with II of different structures at the temperature of 5-100°C, react for 1-48 hours in a heat preservation manner to prepare III;
**[2]** React the compound III with the compound IV under alkaline conditions at the temperature of 5-150°C for 1-48 hours to prepare V;
[3] React the compound V under alkaline conditions at the temperature of 5-100°C for 1-48 hours, connect different side chains VI (Linker, which is equivalent to partial raw material of L in the general Formula 1, to prepare compound VII;
**[4]** React the compound VII and E₃ under alkaline conditions at the temperature of 1-100°C for 1-48 hours to prepare the compound representative example A of the invention. The synthetic scheme is as follows:

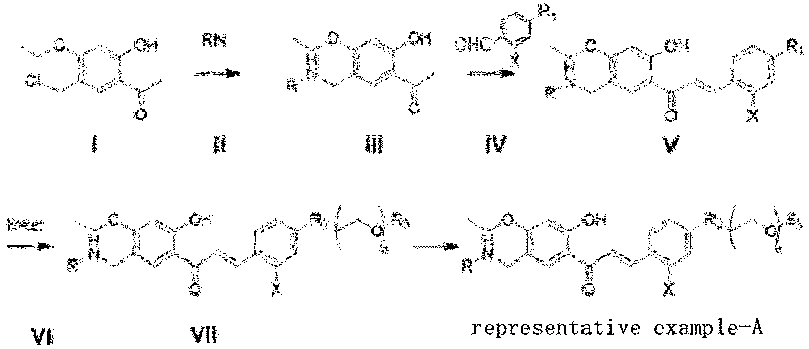

### Pharmaceutical compositions and methods of treatment

The present invention provides a pharmaceutical composition comprising a prophylactically or therapeutically effective amount of a compound of the present invention, or a pharmaceutically acceptable salt, ester, optical isomer, stereoisomer, polymorph, solvate, N-oxide, isotopically labeled compound, metabolite, chelate, complex, clathrate, or prodrug thereof, and a pharmaceutically acceptable carrier, said pharmaceutical composition is preferably in a solid, semi-solid, liquid, or gaseous formulation.

The present invention provides a use of the compound of the invention, or a pharmaceutically acceptable salt, ester, optical isomer, stereoisomer, polymorph, solvate, N-oxide, isotopically-labeled compound, metabolite, chelate, complex, clathrate, or prodrug thereof, or a pharmaceutical composition, in the manufacture of a medicament for the treatment of a SHP2 phosphatase modulated disease.

The SHP2 phosphatase modulated disease is a tumor, such as a solid tumor, a hematological tumor, a malignant tumor, a refractory tumor, a primary or metastatic recurrent tumor, and the like.

The SHP2 phosphatase modulated disease is generally selected from noonan syndrome, leopard syndrome, juvenile myelomonocytic leukemia, neuroblastoma, melanoma, acute myelogenous leukemia, breast cancer, esophageal cancer, lung cancer, colon cancer, brain cancer, neuroblastoma, squamous carcinoma of the head and neck, gastric cancer, anaplastic large cell lymphoma and glioblastoma, but is not limited to these.

The present invention provides a method of treatment of a SHP2 phosphatase modulated disease, comprising administering to a human in need of such treatment an effective amount of a compound of the invention, or a pharmaceutically acceptable salt, ester, optical isomer, stereoisomer, polymorph, solvate, N-oxide, isotopically labeled compound, metabolite, chelate, complex, clathrate, or prodrug thereof, or a pharmaceutical composition of the invention.

The target disease of the method of treatment of the present invention is selected from advanced solid tumors, including primary or metastatic recurrent NSCLC, squamous cell lung carcinoma, adenocarcinoma of lung, squamous carcinoma of the head and neck, gastric cancer, colorectal cancer, pancreatic cancer, and the like; also included are breast cancer, esophageal cancer, lung cancer, colon cancer, brain cancer, neuroblastoma, neuroblastoma, melanoma, squamous carcinoma of the head and neck, anaplastic large cell lymphoma and glioblastoma; malignant hematological tumor diseases include juvenile myelomonocytic leukemia, acute myelogenous leukemia; other diseases related to SHP2 abnormal expression, such as Noonan syndrome, leopard syndrome, type II diabetes, obesity, and the like.

By "pharmaceutically acceptable carrier" herein is meant a diluent, adjuvant, excipient, or vehicle that is administered with the therapeutic agent and which is, within the scope of sound medical judgment, suitable for contact with the tissues of humans and/or other animals without excessive toxicity, irritation, allergic response, or other problem or complication commensurate with a reasonable benefit/risk ratio.

Pharmaceutically acceptable carrier that can be used in the pharmaceutical compositions of the invention include, but are not limited to, sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological saline and aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The composition may also optionally contain minor amounts of wetting agents, emulsifying agents or pH buffering agents. Oral formulations may contain standard carriers such as pharmaceutical grades of mannitol, lactose, starch, magnesium stearate, sodium saccharine, cellulose, magnesium carbonate, and the like. Examples of suitable pharmaceutically acceptable carriers are described in Remington's Pharmaceutical Sciences (1990).

The pharmaceutical compositions of the present invention may act systemically and/or locally. For this purpose, they may be administered by a suitable route, for example by injection (e.g. intravenous, intra-arterial, subcutaneous, intraperitoneal, intramuscular injection, including drip) or transdermally; or by oral, buccal, nasal, transmucosal, topical, in the form of ophthalmic preparations or by inhalation.

For these routes of administration, the pharmaceutical compositions of the present invention may be administered in suitable dosage forms.

Such dosage forms include, but are not limited to, tablets, capsules, lozenges, hard candies, powders, sprays, creams, ointments, suppositories, gels, pastes, lotions, ointments, aqueous suspensions, injectable solutions, elixirs, syrups.

The term "effective amount" as used herein refers to an amount of a compound that, when administered, will alleviate one or more symptoms of the condition being treated to some extent.

The dosing regimen may be adjusted to provide the best desired response. For example, a single bolus may be administered, several divided doses may be administered over time, or the dose may be proportionally reduced or increased as indicated by the exigencies of the therapeutic situation. It is noted that dosage values may vary with the type and severity of the condition being alleviated, and may include single or multiple doses. It is further understood that for any particular individual, the specific dosage regimen will be adjusted over time according to the individual need and the professional judgment of the person administering the composition or supervising the administration of the composition.

The amount of a compound of the invention administered will depend on the subject being treated, the severity of the disorder or condition, the rate of administration, the disposition of the compound, and the judgment of the prescribing physician. Generally, an effective dose is from about 0.0001 to about 50mg per kg body weight per day, e.g., from about 0.01 to about 10 mg/kg/day (single or divided administration). For a 70kg person, this may amount to about 0.007 mg/day to about 3500 mg/day, e.g., about 0.7 mg/day to about 700 mg/day. In some cases, dosage levels no higher than the lower limit of the aforesaid range may be sufficient, while in other cases still larger doses may be employed without causing any harmful side effects, provided that the larger dose is first divided into several smaller doses for administration throughout the day.

The amount or dosage of the compound of the invention in a pharmaceutical composition may be from about 0.01mg to about 1000mg, suitably 0.1 to 500mg, preferably 0.5 to 300mg, more preferably 1 to 150mg, especially 1 to 50mg, for example 1.5mg, 2mg, 4mg, 10mg, 25mg etc.

As used herein, unless otherwise specified, the term "treating" means reversing, alleviating, inhibiting the progression of, or preventing such a disorder or condition, or one or more symptoms of such a disorder or condition, to which such term applies.

As used herein, "individual" includes a human or non-human animal. Exemplary human individuals include human individuals (referred to as patients) with a disease (e.g., a disease described herein) or normal individuals. "non-human animals" in the context of the present invention include all vertebrates, such as non-mammals (e.g., birds, amphibians, reptiles) and mammals, such as non-human primates, livestock and/or domesticated animals (e.g., sheep, dogs, cats, cows, pigs, etc.).

In some embodiments, the pharmaceutical compositions of the present invention may further comprise one or more additional therapeutic or prophylactic agents.

The present invention is described in detail below by way of examples, but is not meant to be limited in any way. Having described the invention in detail and having disclosed specific embodiments thereof, it will be apparent to those skilled in the art that various changes and modifications can be made therein without departing from the spirit and scope thereof.

### Examples

These examples are not provided to limit the scope of the present invention.

The structure of the compound was confirmed by nuclear magnetic resonance spectroscopy ( ¹H NMR) and/or Mass Spectrometry (MS).

Chemical shifts (δ) are given in parts per million (ppm). ¹H NMR were measured on a Bruker 400 or Varian 300 nuclear magnetic instrument using deuterated methanol (CD₃OD), deuterated chloroform (CDCl₃) or hexadeuterated dimethyl sulfoxide (DMSO-d₆) as the test solvent and Tetramethylsilane (TMS) as the internal standard.

The LC-MS measurements were performed on Agilent LC-MS-1110 LC-MS, Agilent LC-MS-6110 LC-MS, Agilent LC-MS-6120 LC-MS (manufacturer: Agilent) or Shimadzu LC-MS 2020.

Preparative high performance liquid chromatography was performed using an MS triggered automatic purification system (Waters), Gilson GX-281(Gilson) or semi-preparative liquid chromatography (Chuangxintongheng LC3000(Ddlsogel, C18, 30mm × 250mm 10 µm)).

The Thin Layer Chromatography (TLC) is carried out by using HUANGHAI HSGF 254(5×20cm) silica gel plate, and the preparative thin layer chromatography is carried out by using a Yantai made GF 254 (0.4-0.5 nm) silica gel plate.

The reaction is detected by Thin Layer Chromatography (TLC) or LC-MS, and the developing agent system used comprises dichloromethane and methanol system, n-hexane and ethyl acetate system, petroleum ether and ethyl acetate system, and is adjusted according to the polarity of the compound to be separated (by adjusting the volume ratio of the solvent or adding triethylamine and the like).

The microwave reaction was carried out using a CEM Discovery Sp (400W, RT^{~}300°C) microwave reactor.

Yucheng Chemical 200-300 mesh silica gel is generally used as the stationary phase of the column chromatography. The system of the eluent comprises a dichloromethane and methanol system and a normal hexane and ethyl acetate system, and the eluent system is adjusted according to the polarity of the compound to be separated (by adjusting the volume ratio of the solvent or adding triethylamine and the like).

The reaction temperature is room temperature (20°C -30°C), unless otherwise specified in the examples.

The reagents used in the examples were purchased from Aldrich Chemical Company, Shanghai Bidepharm technology Co., Ltd., Beijing Greenchem Co., Ltd., Accela ChemBio (Shanghai) Co., Ltd., or IBO technology Co., Ltd., etc.

### Intermediate Synthesis example 1

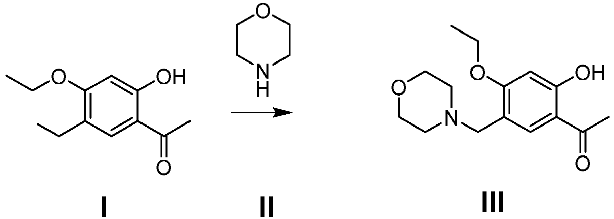

5g of compound I was dissolved in 20 ml of ethyl acetate/dichloromethane, 1g of K₂CO₃ and 5.5 ml of morpholine were added, stirried at room temperature for 24 hours, filtered and washed with ethyl acetate/dichloromethane, and the filtrate is spin-dried. The ester layer was extracted by adding water and ethyl acetate/dichloromethane, acid washed and then neutralized to neutral pH. Extracted with ethyl acetate/dichloromethane. Dried with anhydrous sodium chloride and spin dried to obtain a yellow oily solid. Performed column chromatography purification to obtain a white solid. ¹H NMR (CDCl₃, 300 MHz): δ 12.35 (1H, s, OH), 7.64 (1H, s), 6.42 (1H, s), 3.85 (4H, s), 3.65 (4H, m), 2.52 (7H, m), 1.52 (3H, m). [M+H]⁺ 279.

### Intermediate Synthesis example 2

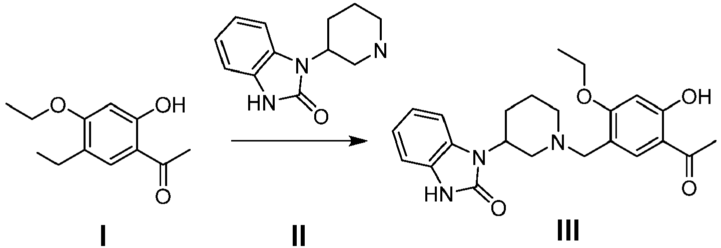

5g of compound I was dissolved in 20 ml of ethyl acetate/dichloromethane, 1g of K₂CO₃ and 7g of compound II were added and stirred at 5-99°C for 0.5-48 hours. Washed with saturated saline solution for three times, dried with sodium carbonate, and rotated. Performed column chromatography to obtain compound III. ¹H NMR (400 MHz, DMSO-d6): δ 13.34 (s,OH), 10.97 (s, 1H), 8.02 (s, 1H), 7.33 (s, 1H), 7.00 (s, 4H), 6.73 (s, 1H), 4.17 (s, 4H), 2.61 (s, 4H), 2.23 (s, 1H), 1.84 (d, J=39.3 Hz, 4H), 1.39 ¨C 1.19 (m, 7H) [M+H]⁺ 409.

### Intermediate Synthesis example 3

Referring to the procedure of example 1, except that a mono-or polysubstituted azaalkyl compound of a different structure from the starting material compound II is reacted with the starting material compound I to give the following compound III.

### Intermediate Synthesis example 4

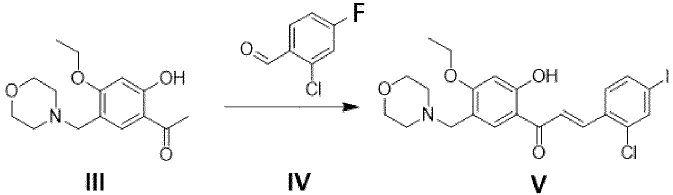

560 mg of compound III was added with 360 mg of compound IV, 800 mg of KOH and 10 ml of ethanol, reacted at room temperature for 1-16 hours, spin dried and added water and ethyl acetate/dichloromethane for extraction, ester layer was dried by anhydrous sodium sulfate, spin dried to obtain a crude product, and performed column chromatography to obtain compound V. [M+H]⁺ 420.4.

### Intermediate Synthesis example 5

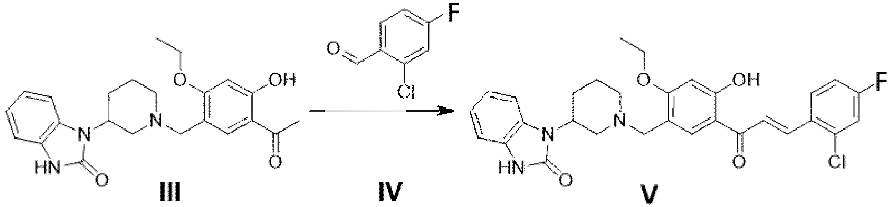

400 mg of compound III was added with 160 mg of compound IV, 500 mg of KOH and 15 ml of ethanol, reacted at room temperature for 0.5-16 hours, spin dried and added water and dichloromethane for extraction, ester layer was dried with anhydrous sodium sulfate, spin dried to obtain a crude product, and performed column chromatography to obtain compound V.

¹H NMR (400 MHz, Chloroform-d) δ 9.18 (s, 1H), 8.16 (d, J =15.6 Hz, 1H), 7.85 (d, J =9.0 Hz, 1H), 7.75 (dd, J =8.8, 5.9 Hz, 1H), 7.55 (d, J =15.6 Hz, 1H), 7.20 (dd, J =8.4, 2.6 Hz, 1H), 7.15 (s, 1H), 7.10-7.00 (m, 5H), 6.50 (d, J =9.1 Hz, 1H), 6.06 (s, 1H), 4.19-4.06 (m, 4H), 3.58 (s, 2H), 3.01 (s, 2H), 2.55 (s, 2H), 2.10-2.02 (m, 2H), 1.49-1.37 (m, 4H) 1.35-1.22 (m, 3H), 0.92-0.81 (m, 2H). ; ESI-MS-m/z): [M+H]⁺ 550.2

### Examples of Synthesis of Compounds

### Example 1 Synthesis of TDC.2001

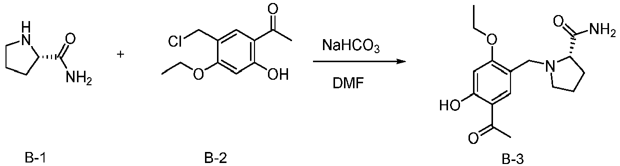

50mg of TDP102197-SM1 was added to 5ml of ultra-dry DMF, followed by 220mg of NaHO₃, 100mg of B-2RT and stirred, monitored by LC-MS until no B-1 remained. The system was added with an appropriate amount of H₂O, extracted with EA, the EA phase was concentrated to dryness to obtain a crude product, which was separated and purified by Per-TLC (DCM:MeOH=20:1) to obtain 120mg of B-3 product (yield: 89.74%, purity: 94.32%), MS: 307.

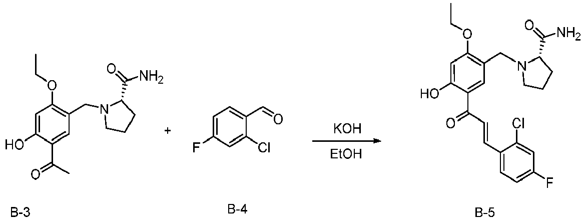

120mg of B-3 was added to 1.5ml of EtOH, followed by 88mg of KOH, 254mg of B-4, stirried at RT and monitored by LC-MS until no B-3 remained. The reaction system was neutralized with AcOH, and then was isolated and purified by Per-TLC (DCM:MeOH=20:1, developed twice) to obtain 275mg (yield: 91.55%) of the product, MS: 448.

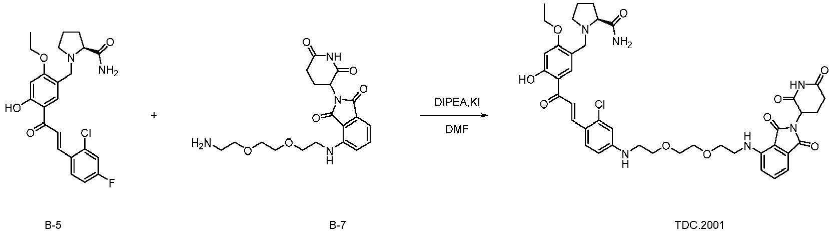

95mg of B-5 was added to 2ml of ultra dry DMF, followed by 269mg of B-7 and 245 mg of DIPEA, raised to 60°C and stirred, monitored by LC-MS until no B-5 remained. The reaction was applied in a thin layer and purified by Per-TLC (DCM:MeOH=20:1) to obtain 30mg of the product (purity: 95.4%), MS: 876. ¹H NMR (400 MHz, DMSO-d6) δ 11.1047 (s, 1H), 9.0103 (m, 1H), 8.3235 (br s, 1H), 8.1298-8.0989 (d, 2H), 7.9523-7.8647 (t, 1H), 7.8320-7.7925 (t, 1H), 7.5516-7.5302 (d, 1H), 7.4647-7.4466 (d, 1H), 6.9990 (s, 1H), 6.7225-6.6998 (d, 1H), 5.1063-5.0741 (d, 1H), 4.3647-4.3417 (t, 2H), 4.2337-4.1998 (t, 2H), 3.8350-3.8121 (t, 2H), 3.7441 (s, 1H), 3.6772-3.6358 (t, H) 3.5610-3.5372 (m, 2H), 3.3262 (s, 2H), 2.9270-2.8351 (q, 2H), 2.7759-2.7316 (d, 2H), 2.5601-2.5255 (t, 4H), 2.0461-1.9713 (m, 2H), 1.6777 (br s, 2H), 1.3899-1.3553 (t, 2H).

### Example 2 Synthesis of TDC.2002

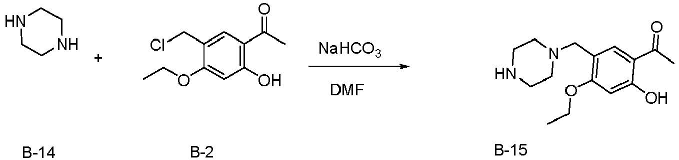

100mg of B-14 was added to 2ml of DMF, followed by 210 mg of NaHO₃ and 106mg of B-2, then the mixture was stirred and three batches were added in parallel. Monitored by LC-MS until no TDP102101-3 remained. Suction filtration, elution of the filter cake with ACN, concentration of the filtrate, separation and purification by Per-TLC (DCM: MeOH=20: 1) gave 333mg (yield: 58.65%, purity: 93%) of the product, MS: 278. ¹H NMR (400 MHz, CDCl₃-*d*) δ 7.70 (t, 1H), 6.48 (s, 1H), 4.08 (q, 2H), 3.75 (d, 2H), 2.84-2.78 (m, 4H), 2.78-2.71 (m, 4H), 2.63 (p, 1H), 1.43 (t, 3H).

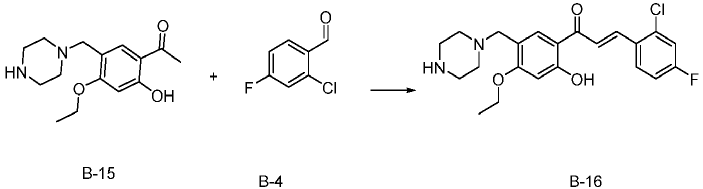

160 mg of B-15 was added to 2ml EtOH, followed by 90 mg of KOH, 280 mg of B4, stirred at RT and monitored by LC-MS until no B-15 remained. The reaction system was neutralized with AcOH and then separated and purified by Per-TLC (DCM:MeOH=20:1) to obtain 136 mg (purity: 90%, yield: 64.72%) of the product, MS: 419.

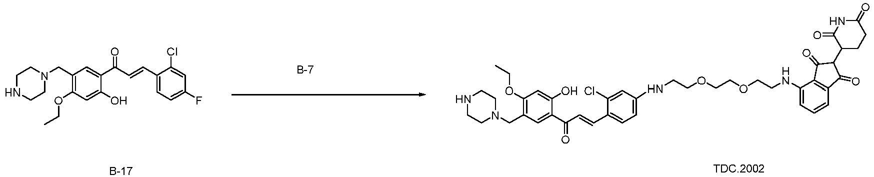

55 mg of B-17 was added into 2ml of ultra-dry DMF, followed by 200 mg of B-7 and 145 mg of DIPEA, raised to 60°C and stirried, monitored by LC-MS until no B-17 remained. The reaction was applied in a thin layer and separated and purified by Per-TLC (DCM:MeOH=20:1) to obtain 13mg (purity: 93.6%) of the product, MS: 802 ¹H NMR (400 MHz, CDCl₃) δ 8.76 (s, 1H), 8.00 (m, 1H), 7.97 (d, 1H), 7.80 (m, 1H), 7.58 (dd, 1H), 7.53 (dd, 1H), 7.44 (m, 1H), 7.37 (m, 1H), 6.99 (m, 1H), 6.90 (m, 1H), 6.69 (m, 1H), 6.56 (s, 1H), 4.63 (tm, 1H), 4.56 (dd, 1H), 4.08 (m, 2H), 3.75 (d, 2H), 3.65 (s, 4H), 3.64 (m, 4H) 3.60-3.49 (m, 2H), 3.50 (s, 1H), 3.49 (m, 1H), 3.42 (m, 2H), 2.84-2.78 (m, 4H), 2.78-2.71 (m, 4H), 2.63 (m, 1H), 2.60-2.48 (m, 2H), 2.16-2.00 (m, 2H), 1.43 (mm, 3H).

### Example 3 Synthesis of TDC.2003

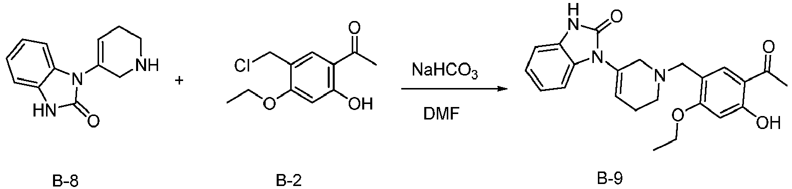

100mg of B-8 was added to 2ml of DMF, followed by 210 mg of NaHO₃ and 106mg of B-2, then the mixture was stirred and three batches were added in parallel. Monitored by LC-MS until no B-8 remained. Suction filtration, elution of the filter cake with ACN, concentration of the filtrate, separation and purification by Per-TLC (DCM:MeOH=20:1) gave 333mg (yield: 58.65%, purity: 93%) of the product, MS: 408. ¹H NMR (400 MHz, CDCl₃). δ.12.88 (s, 1H), 9.74 (s, 1H), 7.69 (d, 1H), 7.13-6.94 (m, 3H), 6.98-6.89 (m, 1H), 6.54 (s, 1H), 6.46 (d, 1H), 4.20 (s, 2H), 4.10 (q, 2H), 3.13 (t, H), 2.58 (s, 3H), 2.25 (t,2H), 2.03-1.97 (m, 2H), 1.34 (t,3H).

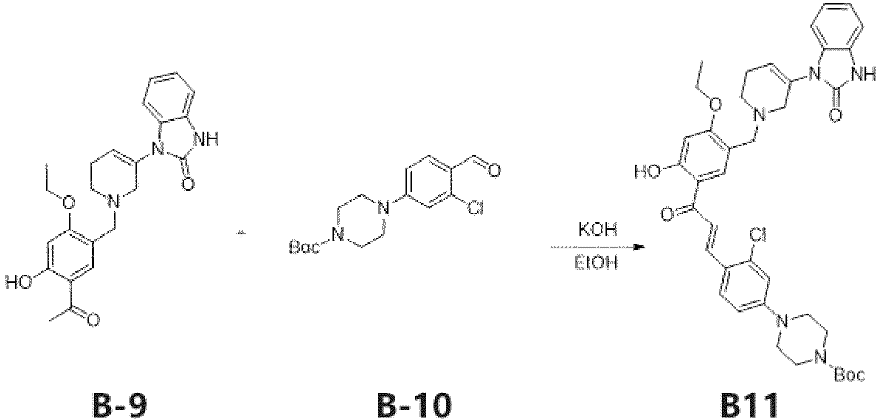

200mg of B-9 was added to 2ml of EtOH, followed by 110mg of KOH, 319mg of B-10, stirried at RT and monitored by LC-MS until no B-9 remained. The reaction system was neutralized with AcOH and then separated and purified by Per-TLC (DCM:MeOH=20:1) to obtain 296mg of the product (purity: 90%, yield: 84.72%), MS: 714. ¹H NMR (400 MHz, CDCl₃). δ 9.05 (s, 1H), 8.20 (d, 1H), 7.86 (d, 1H), 7.68 (d, H), 7.47 (d, 1H), 7.15 (s, 1H), 7.05 (d,3H), 6.89 (d, 1H), 6.80 (dd, 1H), 6.48 (d, H), 6.04 (s, 1H), 5.30 (s, 1H), 4.23-3.91 (m, 5H), 3.58 (t, 7H), 3.29 (t, 4H), 3.13 (s, 1H), 2.95 (s, 2H), 2.51 (s, 2H), 2.08 (s, 2H), 1.49 (s, 9H), 1.42 (t, 3H).

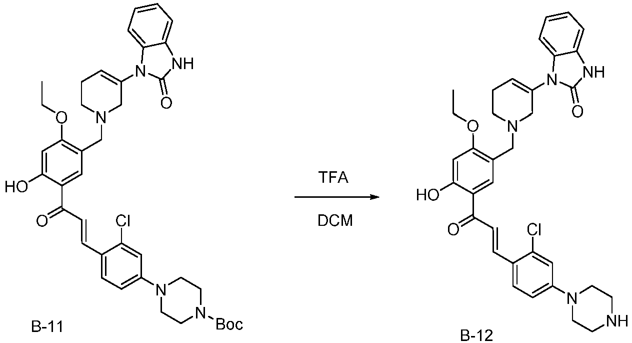

290mg of B-11 was added to 3ml of DCM, followed by 1ml of TFA, stirred at RT and monitored by LC-MS until no B-11 remained. The reaction was concentrated to dryness, neutralized with TEA, and then separated and purified by Per-TLC (DCM:MeOH=20:1, developed twice) to obtain 150mg of the product (yield: 58.94%, purity: 98%), MS: 614.

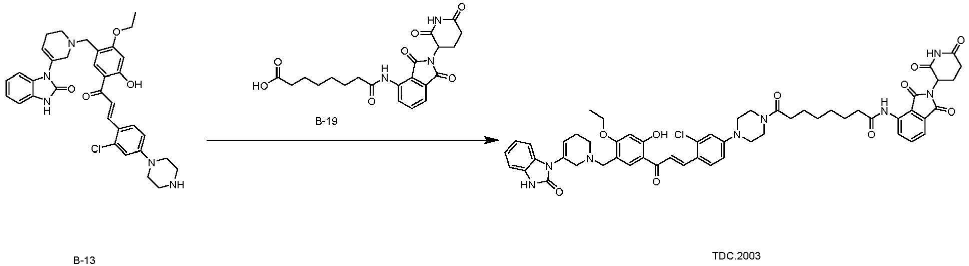

200mg of B-13 was added into 2ml DMF, followed by 4eq DIPEA, 122 mg of B-19 were added, stirred at RT and Monitored by LC-MS until no B-13 remained. Purified by Pre-TLC with DCM:MeOH=10:1 to obtain 180mg (purity: 92%) of the product. MS:1025; ¹H NMR (400 MHz, CDCl₃) δ 9.51 (s, 1H), 8.11 (m, 1H), 7.97 (d, 1H), 7.80 (m, 1H), 7.72-7.60 (m, 3H), 7.58-7.49 (m, 2H), 7.42 (m, 1H), 7.27 (m, 1H), 7.16 (m, 1H), 6.97 (d, 1H), 6.80 (m, 1H), 6.56 (s, 1H) 5.50 (m, 1H), 5.41 (m, 1H), 4.08 (m, 2H), 3.85 (d, 2H), 3.55-3.48 (m, 4H), 3.45 (m, 2H), 3.33-3.26 (m, 4H), 2.94-2.81 (m, 3H), 2.64-2.49 (m, 2H), 2.41 (m, 2H), 2.31 (m, 2H), 2.15-2.03 (m, 3H), 1.71-1.56 (m, 4H), 1.43 (mm, 3H), 1.41-1.27 (m, 4H).

### Example 4 Synthesis of TDC. 2004

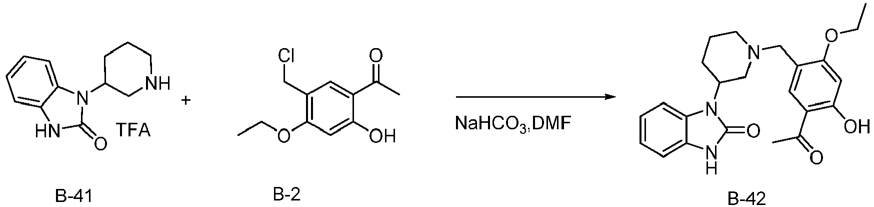

125 mg of B41, 200mg of sodium bicarbonate, 5ml of DMF were added into a single-neck bottle, added 130 mg of B-2, stirried at room temperature for 3 hours, monitored reaction progress by LC-MS, filtered the reaction solution after the reaction, separated the collected filtrate by thin layer chromatography, developing agent DCM:MeOH (20:1), MS:410.20

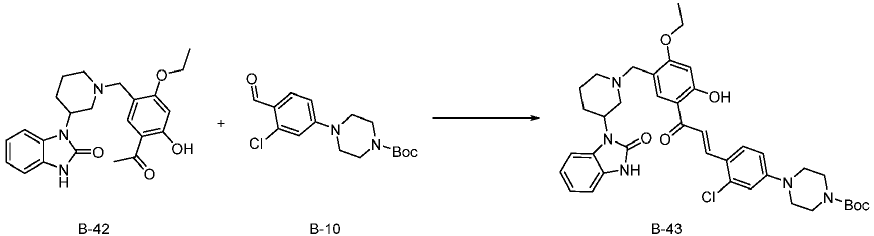

100 mg of B-42 was added to 2ml of EtOH, followed by 110mg of KOH, 110mg of B-10, stirried at RT and monitored by LC-MS until no B-42 remained. Separated and purified by Per-TLC (DCM:MeOH=20:1, developed twice) to obtain 296mg of the product (purity: 90%, yield: 84.72%), MS: 716. MS: 716.34

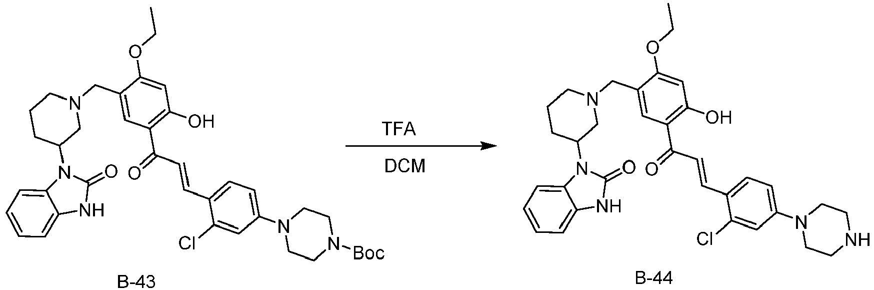

100 mg of B-43 was added to 1ml of DCM, followed by 1ml of TFA, stirred at RT and monitored by LC-MS until no B-43 remained. The reaction system was concentrated to dryness, neutralized with TEA, and then separated and purified by Per-TLC (DCM:MeOH=20:1, developed twice) to obtain 52 mg of the product (yield: 58.94%, purity: 98%), MS: 616.

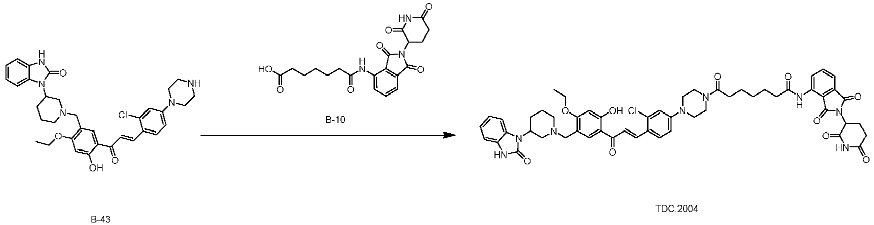

200mg of B-43 was added to 2ml of DMF, followed by 110mg of DIPEA, 319mg of B-10, stirred at RT and monitored by LC-MS until no B-43 remained, Purified by Pre-TLC, DCM:MeOH=10:1 to obtain 180mg (purity: 92%) of the product MS:1013.5; ¹H NMR (400 MHz, CDCl₃) δ 9.51 (s, 1H), 8.11 (m, 1H), 7.97 (d, 1H), 7.80 (m, 1H), 7.70-7.60 (m, 2H), 7.58-7.49 (m, 2H), 7.46-7.39 (m, 1H), 7.42-7.32 (m, 1H), 7.21-7.08 (m, 2H), 6.97 (d, 1H), 6.80 (m, 1H) 6.56 (s, 1H), 5.41 (m, 1H), 4.50 (m, 1H), 4.08 (m, 2H), 3.85 m, 1H), 3.76 (m, 1H), 3.55-3.48 (m, 4H), 3.33-3.26 (m, 4H), 3.13 (m, 1H), 3.02 (m, 1H), 2.92-2.81 (m, 1H), 2.77-2.49 (m, 4H), 2.41 (m, 2H), 2.31 (m, 2H), 2.15-2.03 (m, 1H), 1.96-1.83 (m, 2H), 1.77-1.46 (m, 9H), 1.43 (m, 3H).

### Example 5 Synthesis of TDC.2005

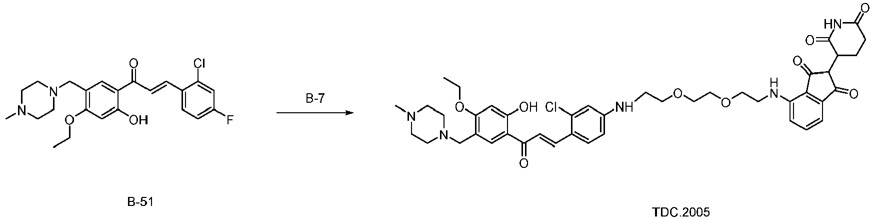

45 mg of B-51 was added into 2ml of ultra-dry DMF, followed by 100 mg of B-7 and 145 mg of DIPEA, raised to 60°C and stirried, monitored by LC-MS until no B-51 remained. The reaction was applied in a thin layer and separated and purified by Per-TLC (DCM:MeOH =20:1) to obtain 34 mg (purity: 92.1%), MS: 816 ¹H; NMR (400 MHz, CDCl₃) δ 8.76 (s, 1H), 8.00 (d, 1H), 7.97 (m, 1H), 7.80 (m, 1H), 7.58 (d, 1H), 7.53 (d, 1H), 7.44 (m, 1H), 7.37 (m, 1H), 6.99 (m, 1H), 6.90 (dd, 1H), 6.69 (m, 1H), 6.56 (s, 1H), 4.63 (m, 1H), 4.56 (d, 1H), 4.08 (m, 2H), 3.75 (d, 2H), 3.65 (s, 4H), 3.64 (m, 4H) 3.60-3.49 (m, 2H), 3.50 (s, 1H), 3.49 d, OH), 3.42 (m, 2H), 2.61-2.47 (m, 6H), 2.50-2.41 (m, 4H), 2.16-2.00 (m, 2H), 1.43 (m, 3H).

### Example 6 Synthesis of TDC.2006

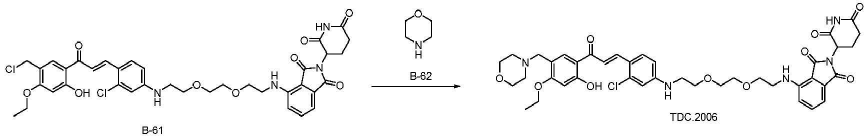

75 mg of B-61, 156 mg of B-62, 200mg of sodium bicarbonate and 5ml of DMF were added into a single-mouth bottle for reaction for 2 hours, and then column chromatography was carried out to obtain 20 mg of the product (purity: 88%). MS: 803. ¹H NMR (400 MHz, CDCl₃) δ 9.51 (s, 1H), 7.97 (d, 1H), 7.80 (m, 1H), 7.71 (m, 1H), 7.61-7.49 (m, 3H), 7.45 (m, 1H), 7.09 (m, 1H), 6.90 (d, 1H), 6.69 (m, 1H), 6.56 (s, 1H), 5.41 (m, 1H), 4.63 (mm, 1H), 4.08 (m, 2H), 3.75 (d, 2H), 3.70 (s, 1H), 3.72-3.66 (m, 3H), 3.65 (s, 4H), 3.64 (m, 4H), 3.50 (m, 2H), 3.42 (m, 2H), 2.92-2.81 (m, 1H), 2.67-2.59 (m, 4H), 2.63-2.49 (m, 2H), 2.15-2.03 (m, 1H), 1.43 (m, 3H).

### Example 7 Synthesis of TDC.2007

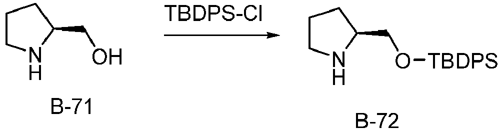

1.4 g of imidazole, 1 g of B-71 and 12ml of DCM were added into a 50ml single-mouth bottle, cooled to below 0°C and started stirring, 3.3g of TBDPS-Cl was added dropwise to the reaction system, kept the temperature and stirried for 3 hours after adding was finished, monitored by LC-MS and added 10ml of saturated sodium bicarbonate solution for washing after the reaction was finished, collected the organic phase and added 4.0g of silica gel for stirring, and purified by column chromatography to obtain a colorless oily substance with the yield of 55.9%. MS: 340.2

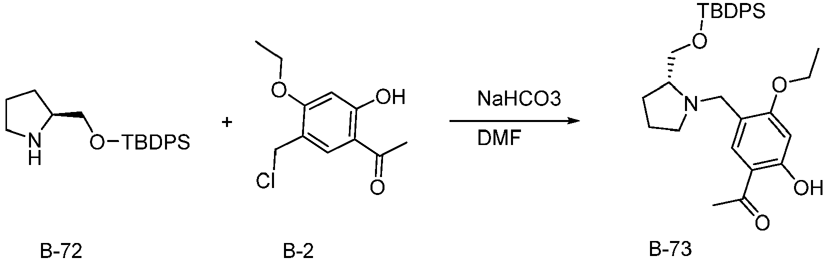

220 mg of B-72, 440 mg of B-2, 300 mg of sodium bicarbonate and 6 ml of DMF were added into a single-mouth bottle, stirried overnight at room temperature, filtered under reduced pressure after the reaction was finished, purified the collected organic phase by medium pressure preparation, collected 90% acetonitrile elution part, evaporated to dryness under reduced pressure, and confirmed the liquid quality to obtain a yellow oily substance with the yield of 63.6%. MS: 532.29

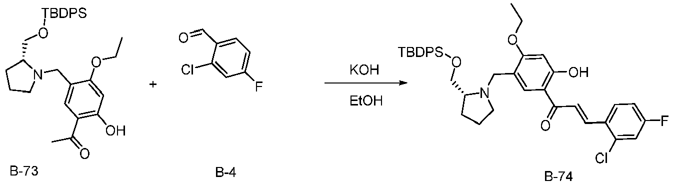

300 mg of B-73, 366mg of B-10, 127 mg of KOH and 5ml of EtOH were added into a 25ml single-mouth bottle, stirred overnight at room temperature and the progress of the reaction was monitored by LC-MS. And after the reaction was finished, evaporated the reaction system to dryness under reduced pressure, added 15ml each of EA and water, then added a few drops of acetic acid, adjusted the pH of water phase to about 6, separated and collected the organic phase, prepared and separated the upper thin layer, developing agent PE:EA=1:1, collected the yellow layer with the Rf value of about 0.5. Concentrated to dryness to obtain a yellow oil with yield of 72.9%. MS: 672

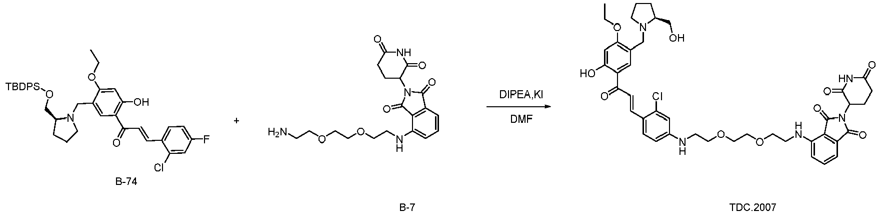

200 mg of B-77, 5ml of 1MTBAF tetrahydrofuran solution were weighed, stirred at room temperature for 2 hours, the reaction solution was directly subjected to preparative separation at medium pressure, and gradient elution was carried out with acetonitrile/water (containing 0.1% formic acid) to obtain 93 mg of the product. MS: 818.3 HNMR: ¹H NMR (400 MHz, CDCl₃) δ 9.51 (s, 1H), 7.97 (d, 1H), 7.80 (m, 1H), 7.71 (m, 1H), 7.61-7.49 (m, 3H), 7.45 (m, 1H), 7.09 (m, 1H), 6.90 (d, 1H), 6.69 (m, 1H), 6.56 (s, 1H), 5.41 (m, 1H), 4.63 (m, 1H), 4.08 (m, 2H), 3.79 (m, 1H), 3.70 (m, 1H), 3.70-3.58 (m, 10H), 3.50 (m, 2H), 3.48-3.41 (m, 1H), 3.45-3.38 (m, 2H), 2.92-2.81 (m, 2H) 2.77 (m, 1H), 2.74-2.66 (m, 1H), 2.64-2.49 (m, 2H), 2.15-2.03 (m, 1H), 1.82-1.60 (m, 4H), 1.43 (m, 3H).

### Example 8 Synthesis of TDC2008

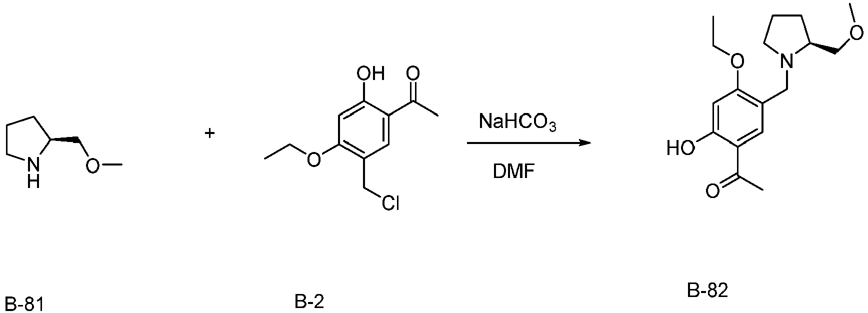

200mg of B-81 was added to 4ml of DMF, 880mg of NaHO₃ and 400mg of B-2 were added, then stirred at RT. Monitored by LC-MS until no B-81 remained. Suction filtration, ACN rinsing of the filter cake, concentration of the filtrate, separation and purification by Per-TLC (DCM:MeOH=20:1) were carried out to abtain 334mg of the product (purity: 82%), MS: 308.

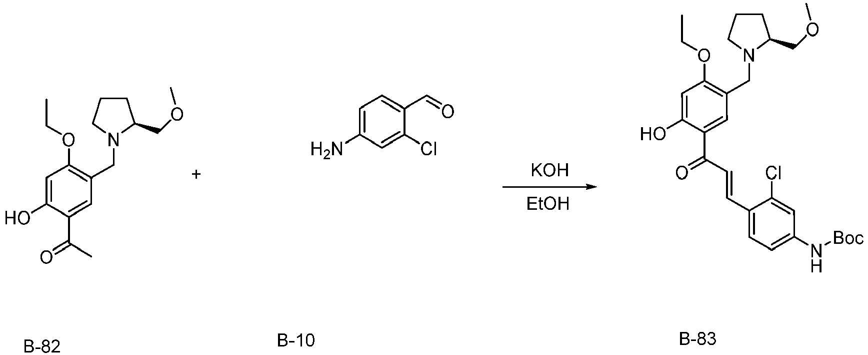

303mg of B-82 was added to 4ml of EtOH, followed by 221mg of KOH and 640mg of B-10, stirred at RT and monitored by LC-MS until no B-82 remained. The reaction system was neutralized with AcOH and then isolated and purified by Per-TLC (DCM:MeOH=20:1) to obtain 446mg of the product (purity: 90%, yield: 73.79%), MS: 554.

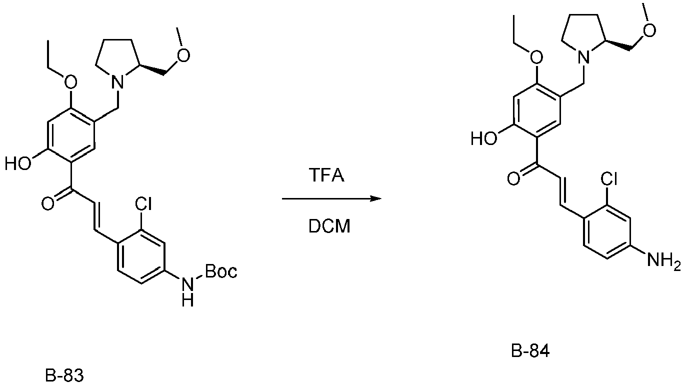

446mg of B-83 was added to 4ml of DCM, followed by 2ml of TFA, stirred at RT and monitored by LC-MS until no B-83 remained. The reaction system was concentrated to dryness, neutralized with TEA and purified by Per-TLC (DCM:MeOH=20:1) to obtain an oil (purity: 90%) which was charged to the next step as 0.726mmol, MS: 445.

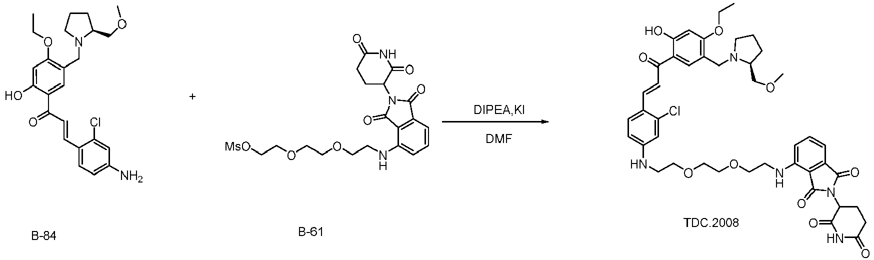

0.726mmol of B-84 was added to 3ml of DMF, followed by 1055mg of B-61 and 938mg of DIPEA, raised to 80°C and stirred, and monitored by LC-MS until no TDP102215-3 remained. The system was separated and purified by Per-TLC (DCM:MeOH=20:1, developed twice) to obtain a crude product, which was separated and purified by semi-preparative reverse phase separation followed by Per-TLC (DCM:MeOH=20:1) to obtain 24mg (purity: 89.07%), MS: 832. ¹H NMR (400 MHz, Chloroform-*d*) δ 9.51 (s, 1H), 7.97 (dd, 1H), 7.80 (m, 1H), 7.71 (m, 1H), 7.61-7.49 (m, 3H), 7.45 (m, 1H), 7.09 (m, 1H), 6.90 (d, 1H), 6.69 (m, 1H), 6.56 (s, 1H), 5.41 (m, 1H), 4.63 (mm, 1H), 4.08 (m, 2H), 3.81 (mm, 1H), 3.70 (m, 1H), 3.65 (s, 4H), 3.67-3.60 (m, 4H), 3.50 (m, 2H), 3.42 (m, 2H), 3.39-3.27 (m, 2H), 3.31 (s, 3H) 2.92-2.79 (m, 3H), 2.69 (m, 1H), 2.64-2.49 (m, 2H), 2.15-2.03 (m, 1H), 1.82-1.73 (m, 1H), 1.77-1.69 (m, 2H), 1.73-1.62 (m, 1H), 1.43 (m, 3H).

### Example 9 Synthesis of TDC2009

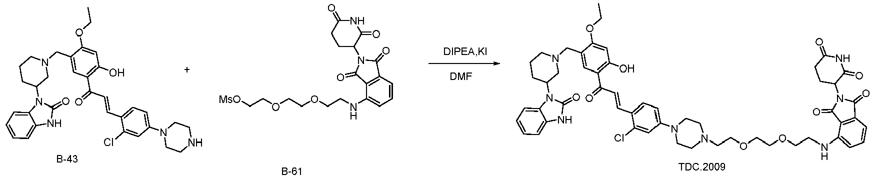

140 mg of B-43, 330 mg of B-61, 300 mg of DIPEA and 3ml of DMF were added into a single-neck round-bottom flask, kept the temperature at 80°C and stirried for 5-6 hours, added 15ml each of EA and water after the reaction was finished, washed by water twice, dried the organic phase by using anhydrous sodium sulfate, prepared and separated the crude product obtained by decompression and concentration by using medium pressure, and performed gradient elution on acetonitrile/water under the reverse phase condition to obtain the product. MS: 1003, HNMR: ¹H NMR (400 MHz, CDCl₃) δ 9.51 (s, 1H), 7.97 (d, 1H), 7.80 (m, 1H), 7.71 (t m, 1H), 7.58-7.49 (m, 3H), 7.45 (m m 1H), 7.09 (m, 1H), 6.97 (m, 1H), 6.80 (m, 1.9 Hz, 1H), 6.56 (s, 1H), 5.41 (m, 1H), 4.08 (m, 2H), 3.81 (m, 1H), 3.70 m, 1H), 3.70-3.60 (m, 6H), 3.58-3.46 (m, 4H), 3.39-3.23 (m, 9H), 2.92-2.74 (m, 7H), 2.73-2.64 (m, 3H), 2.64-2.49 (m, 2H), 2.15-2.03 (m, 1H), 1.81-1.73 (m, 1H), 1.77-1.69 (m, 2H), 1.73-1.62 (m, 1H), 1.43 (m, 3H).

### Example 10 Synthesis of TDC2010

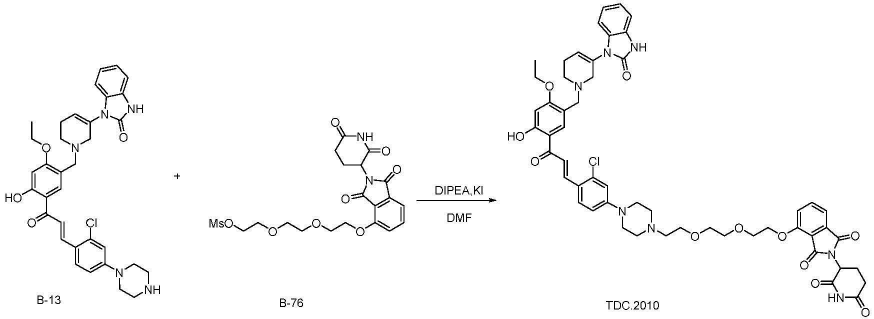

57mg of B-13 was added to 2ml of DMF, followed by 133mg of B-76 and 119mg of DIPEA, raised to 80°C and stirred, monitored by LC-MS until no B-13 remained. The reaction mixture was concentrated to dryness under reduced pressure at 40°C and dissolved in an appropriate amount of DCM, and then the mixture was applied to a thin layer and subjected to Per-TLC separation and purification (DCM:MeOH=20:1) to obtain 42mg of the product, and further subjected to semi-preparative reverse phase separation and purification to obtain 8mg (purity: 92.9%), MS: 1002. ¹H NMR (400 MHz, DMSO-*d₆*) δ 11.10 (s,1H), 10.93 (s,1H), 8.34 (s,1H), 8.26 (d, 1H), 8.13-8.03 (m, 2H), 7.91-7.73 (m, 2H), 7.54 (d, H), 7.45 (m, 1H), 6.98 (s, 6H), 6.67 (d, H), 5.89 (s,1H), 5.08 (m, 2H), 4.35 (m, 2H), 4.16 (m, 2H), 3.82 (m, 3H), 3.77-3.61 (m, 5H), 3.60-3.50 (m, 5H), 2.88 (s,1H), 2.71 (d, 2H), 2.64-2.50 (m, 2H) 2.29 (s, 3H), 2.11-1.95 (m, 2H), 1.38-1.26 (m, 4H), 1.23 (s, 5H).

### Example 11 Synthesis of TDC2011

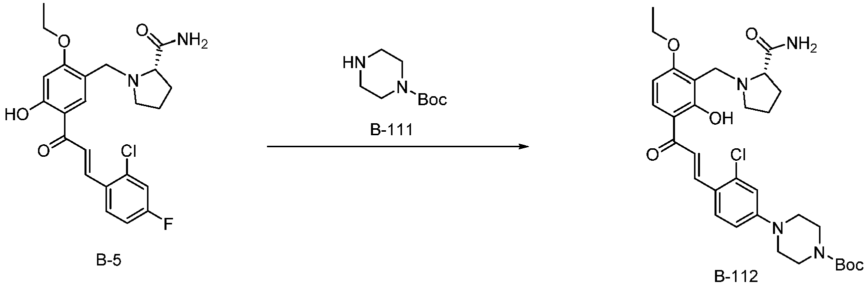

100 mg of B-5 was added to 3ml of DMF, followed by 1.5ml of DIPEA, stirred at 60°C and monitored by LC-MS until no B-55 remained. The reaction system was concentrated to dryness, neutralized with TEA, then separated and purified by Per-TLC (DCM:MeOH=10:1) to obtain 46 mg of the product (yield: 40.12%, purity: 77%).

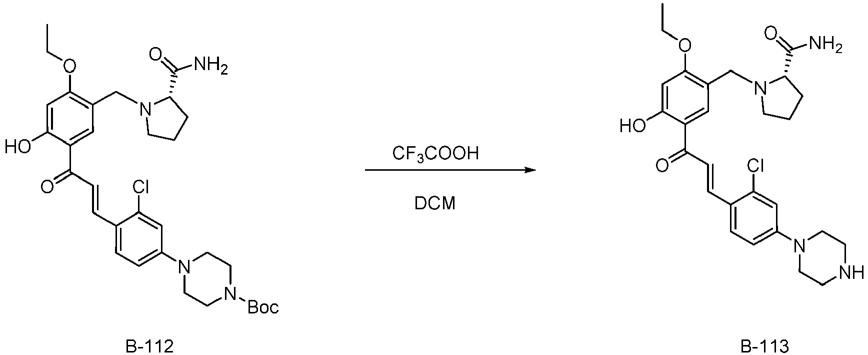

275mg of B-112 was added to 3ml of DCM, followed by 1.5ml of CF₃COOH, stirred at RT, and monitored by LC-MS until no B-112 remained. The reaction system was concentrated to dryness, neutralized with TEA, and then separated and purified by Per-TLC (DCM:MeOH=20:1) to obtain 144mg of the product (yield: 62.69%, purity: 93%), MS: 513.

¹H NMR (400 MHz, DMSO-d6) δ 14.0677 (br s, 1H), 8.9402 (br s, 2H), 8.3977-8.3751(d, 1H), 8.2115-8.1890 (d, 1H), 8.1500-8.1118 (d, H), 7.9709-7.9329 (d,1H), 7.1450-7.1388 (d, 1H), 7.0978-7.0755 (d, 1H), 6.7485-6.7257 (d, 1H), 4.2445-4.2081 (m, 2H), 3.9016 (br s, 2H), 3.7234(s, 1H), 3.6013-3.5757 (m, 4H), 3.4525(s, 1H), 3.2387-3.2123 (m, 4H), 3.12-3.0698 (m, 4H), 1.7394 (br s, 2H), 1.4043-1.3697 (m, 3H).

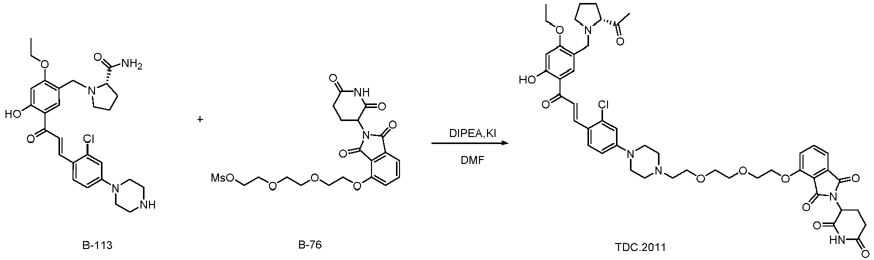

95mg of B-113 was added to 2ml of DMF, followed by 269mg of B-76 and 239mg of DIPEA, raised to 80°C and stirred, monitored by LC-MS until no B-113 remained. The reaction was applied in a thin layer and separated and purified by Per-TLC (DCM:MeOH=20:1) to obtain 30mg of the pruduct (purity: 12.8%+85%), MS: 901. 1H NMR (400 MHz, DMSO-d6) δ 11.1047 (s, 1H), 8.3235 (br s, 1H), 8.1298-8.0989 (d, 2H), 7.9523-7.8647 (m, 1H), 7.8320-7.7925 (m, 1H), 7.5516-7.5302 (d, 1H), 7.4647-7.4466 (d, 1H), 6.9990 (s, 1H), 6.7225-6.6998 (d, 1H), 5.1063-5.0741 (d, 1H), 4.3647-4.3417 (m, 2H), 4.2337-4.1998 (m, 2H), 3.8350-3.8121 (m, 2H), 3.7441 (s, 1H), 3.6772-3.6358 (m, 2H), 3.5610-3.5372 (m, 2H), 3.3262 (s, 10H), 2.9270-2.8351 (m, 2H), 2.7759-2.7316 (d, 2H), 2.5601-2.5255 (m, 4H), 2.0461-1.9713 (m, 2H), 1.6777 (br s, 2H), 1.3899-1.3553 (m, 2H).

### Example 12 Synthesis of TDC2012

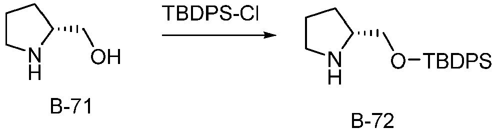

1.4 g of imidazole, 1 g of B-71 and 12ml of DCM were added into a 50ml single-mouth bottle, cooled to below 0°C, started stirring, weighed 3.3g ofTBDPS-CI and added dropwise into the reaction system, kept the temperature and stirried for 3 hours after the dropwise adding was finished, monitored by LC-MS, added 10ml of saturated sodium bicarbonate solution after the reaction was finished, washing, collected the organic phase, added 4.0g of silica gel for stirring, and purified by column chromatography to obtain a colorless oily substance with the yield of 55.9%. MS 340.2

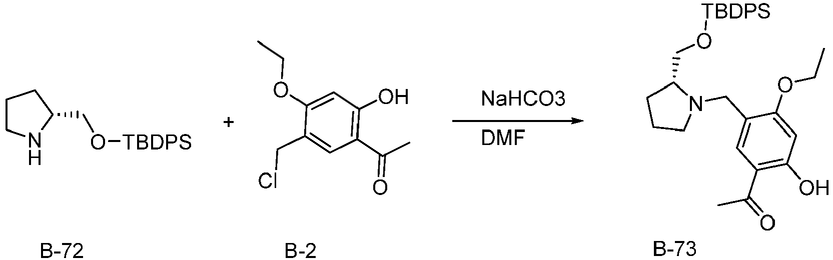

220 mg of B-72, 440 mg of B-2, 300 mg of sodium bicarbonate and 6 ml of DMF were added into a single-mouth bottle, stirried overnight at room temperature, filtered under reduced pressure after the reaction was finished, purified the collected organic phase by medium pressure preparation, collected 90% acetonitrile elution part, evaporated to dryness under reduced pressure, and confirmed the liquid quality to obtain a yellow oily substance with the yield of 63.6%. MS: 532.29

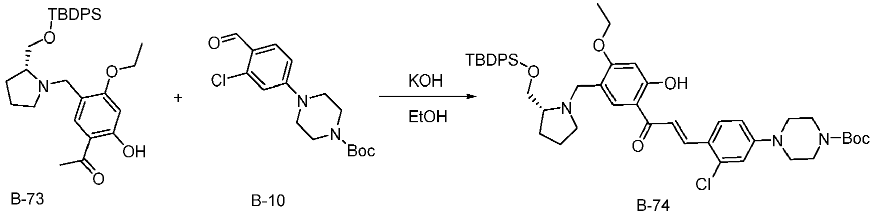

300 mg of B-73, 366mg of B-10, 127 mg of KOH and 5ml of EtOH were added into a 25ml one-Neck flask, stirred overnight at room temperature and the progress of the reaction was monitored by LC-MS. after the reaction was finished, evaporated the reaction system to dryness under reduced pressure, added 15ml each of EA and water for liquid separation, added a few drops of acetic acid, adjusted the pH of the water phase to about 6, separated and collected the organic phase, prepared and separated the upper thin layer, developing agent PE:EA=1:1, collected the yellow layer with the Rf value of about 0.5. Concentrated to dryness to obtain a yellow oil with yield of 72.9% MS: 838.2

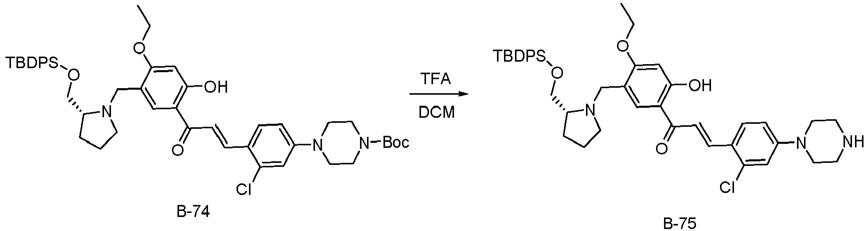

340 mg of B-74, 2ml of TFA and 6 ml of DCM were added into a single-mouth bottle, stirried the mixture for 3 hours at room temperature, evaporated to dryness under reduced the pressure after the reaction was finished, diluted the residue with a small amount of DCM, added TEA to neutralize the residual trifluoroacetic acid, directly prepared and separated the upper thin layer, developed by DCM/MeOH=30:1, collected the orange yellow layer with the Rf value of 0.1, confirmed by LC-MS, concentrated under the reduced pressure to obtain a yellow oily substance, 241mg , with yield of 80 %, MS: 738.3548

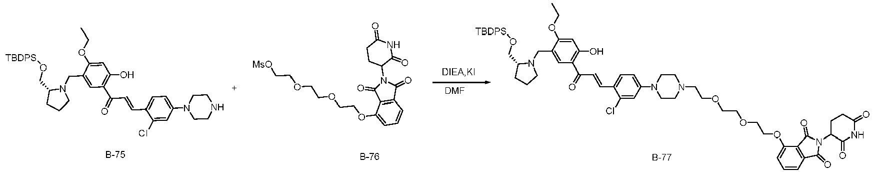

240 mg of B-75, 230 mg of B-10, 400 mg of DIPEA and 5ml of DMF were added into a 50ml single-neck bottle, stirred overnight at 80°C, after the reaction liquid is cooled down, prepared and separated the upper thin layer, developing agent DCM/MeOH=15:1, collected the yellow layer with the Rf value of about 0.5, filtered and concentrated to obtain a yellow oily substance 170 mg, with the yield of 54.6%. LMS: 1126.38

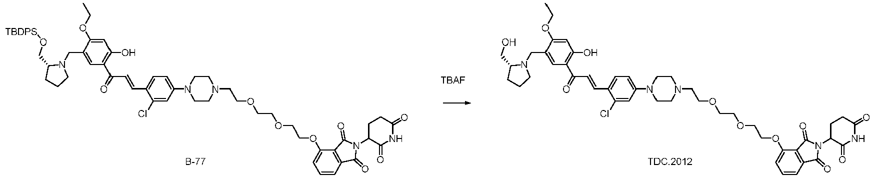

200 mg of B-77, 5ml of 1MTBE tetrahydrofuran solution were stirred at room temperature for 2 hours, the reaction mixture was directly subjected to preparative separation under medium pressure, and performed gradient elution with acetonitrile/water (containing 0.1% formic acid) to obtain the product as a brown oily substance 100 mg with a purity of 95%. yield 61%. MS: 888.36 HNMR: 1H NMR (400 MHz, DMSO-d6) δ 11.10 (s, 1H), 8.30 (s, 2H), 8.15 (d, H), 8.10-7.99 (m, 1H), 7.82 (dd, 8.7 Hz, 2H), 7.54 (d, 1H), 7.45 (d, 1H), 7.05-6.93 (m, 2H), 6.64 (d, 1H), 5.08 (dd, 1H), 4.39-4.31 (m, 2H), 4.15 (s, 2H), 3.92 (d, 1H), 3.82 (d, 1H), 3.82 (s, 2H), 3.70-3.60 (m, 3H), 3.59-3.50 (m, 5H), 3.30 (s, 5H), 3.16 (s, 2H) 3.16 (d, 1H), 2.96-2.76 (m, 1H), 2.63-2.50 (m, 3H), 2.06-1.96 (m, 2H), 1.81 (s, 1H), 1.57 (s, 4H), 1.34 (dt, 3H), 1.24 (s, 4H), 0.94 (t, 2H).

### Example 13 TDC2013

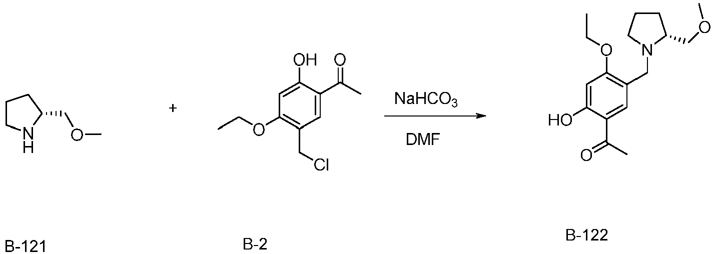

200mg of B-121 was added to 4ml of DMF, followed by 880mg of NaHO₃ and 400mg of B-2, then stirred at RT. Monitored by LC-MS until no B-121 remained. Suction filtered, rinsed the filter cake by ACN, concentrated the filtrate and the separated and purified by Per-TLC (DCM:MeOH=20:1) to obtain 334mg of the product (yield: 62.63%, purity: 82%), MS: 308.

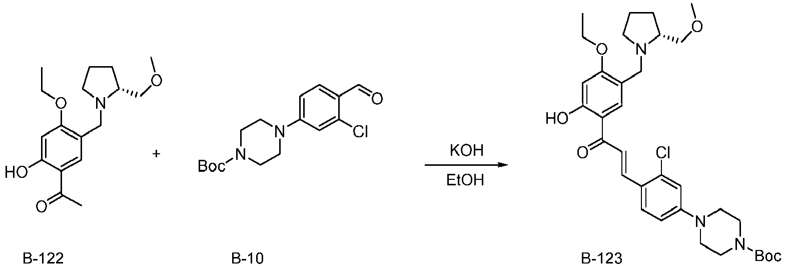

303mg of B-122 was added to 4ml of EtOH, followed by 221mg of KOH, 640mg of B-10, stirried at RT and monitored by LC-MS until no B-122 remained. The reaction system was neutralized with AcOH and then separated and purified by Per-TLC (DCM:MeOH=20:1) to obtain 446mg of the product (purity: 88%, yield: 73.79%), MS: 614.

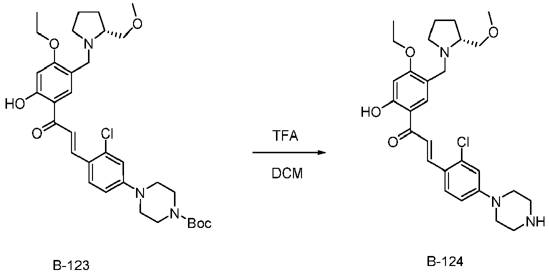

446mg of B-123 was added into 4ml of DCM, followed by 2ml of TFA, stirried at RT, and monitored by LC-MS until no B-123 remained. The reaction system was concentrated to dryness, neutralized with TEA and purified by Per-TLC (DCM:MeOH=20:1) to obtain an oily substance (purity: 99%) which was charged to the next step as 0.726mmol, MS: 514.

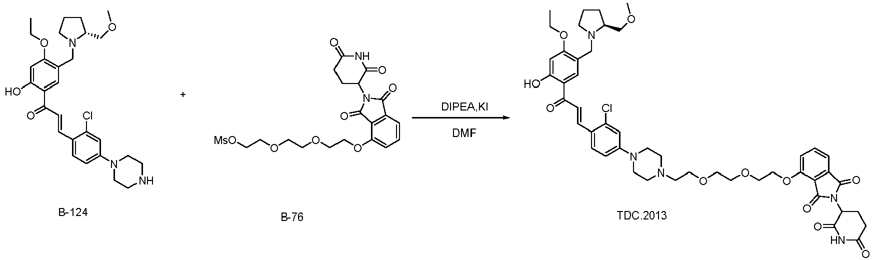

0.726mmol of B-124 was added to 3ml of DMF, followed by 1055mg of B-76 and 938mg of DIPEA, heated to 770°C and stirred, monitored by LC-MS until no B-124 remained. The system was separated and purified by Per-TLC (DCM:MeOH=20:1, developed twice) to obtain the crude product, which was purified by semi-preparative reverse phase separation and then separated and purified by Per-TLC (DCM:MeOH=20:1) to obtain 342 mg (purity: 547.07%), MS: 902. ¹H NMR (400 MHz, DMSO-*d*₆) δ.11.11 (s, 1H), 8.41 (s, 1H), 8.17 (d, 1H), 8.10-8.00 (m, 2H), 7.89-7.76 (m, 2H), 7.54 (d, 1H), 7.45 (d, 1H), 6.97 (d, 2H), 6.64 (d, 1H), 5.08 (m, 1H), 4.35 (m, 2H), 4.15 (m, 2H), 3.94 (d, 1H), 3.82 (mm, 2H), 3.70-3.57 (m, 3H), 3.59-3.44 (m, 6H), 3.26 (s, 3H), 3.18 (m, 1H), 2.96-2.75 (m, 1H), 2.80 (s, 1H) 2.71 (s, 1H), 2.63-2.50 (m, 2H), 2.37-2.27 (m, 1H), 2.10-1.96 (m, 2H), 1.81 (d, 1H), 1.62-1.49 (m, 4H), 1.38 (m, 3H), 1.33-1.20 (m, 5H), 0.85 (m, 1H)

### Example 14 Synthesis of TDC2014

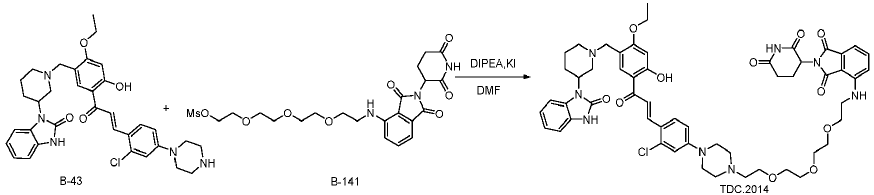

140 mg of B-43, 350mg of B-141, 300 mg of DIPEA and 10ml of DMF were added into a single-neck round bottom flask, kept the temperature at 80°C and stirried for 5-6 hours, after the reaction was finished, added 15ml each of EA and water for liquid separation, washed with water twice, dryied the organic phase with anhydrous sodium sulfate, prepared and separated the crude product obtained by decompression concentration under medium pressure, and performed gradient elution with acetonitrile/water under reverse phase condition to obtain 155 mg of the product with a purity of 91.1% yield 64%. MS: 1047. HN MR: ¹H NMR (400 MHz, CDCl₃) δ 9.51 (s, 1H), 7.97 (d, 1H), 7.80 (m, 1H), 7.71 (m, 1H), 7.58-7.49 (m, 3H), 7.49-7.39 (m, 2H), 7.39-7.32 (m, 1H), 7.21-7.06 (m, 3H), 6.97 (d, 1H), 6.80 (m 1H), 6.56 (s, 1H), 5.41 (m, 1H), 4.50 (m, 1H), 4.08 (m, 2H), 3.85 (mm, 1H), 3.76 (m, 1H), 3.71-3.61 (m, 10H), 3.58-3.46 (m, 4H), 3.30-3.23 (m, 4H) 3.13 (m, 1H), 3.02 (m, 1H), 2.92-2.81 (m, 1H), 2.81-2.71 (m, 4H), 2.76-2.63 (m, 4H), 2.63-2.49 (m, 2H), 2.15-2.03 (m, 1H), 1.96-1.83 (m, 2H), 1.77-1.62 (m, 2H), 1.43 m, 3H).

### Example 15 Synthesis of TDC2015

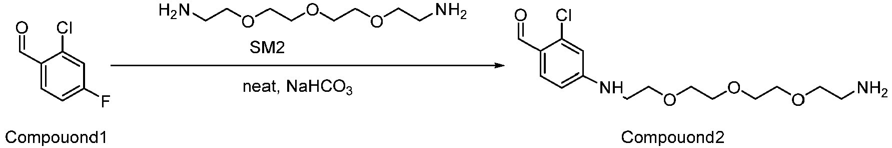

Compound 1 was added with 70 ml of SM2 and 8g of NaHO₃, heated to 120°C and continued to react for 36 h, added water to stop the reaction, the whole reaction was under Ar protection. Extracted with ethyl acetate, dried with NaSO₄, and spin-dried. Performed normal phase column chromatography with EA:PE=1:1 to obtain 16 g of the product with a purity of 95%, yield: 76%, MS: 333. ¹H NMR (400 MHz, CDCl₃) 10.32 (s, 1H), 9.51 (s, 1H), 7.88 (d, 1H), 6.97 (d, 1H), 6.92 (m, 1H), 4.21 (m, 2H), 3.90-3.85 (m, 2H), 3.74-3.65 (m, 19H), 3.61 (m, 5H), 3.26 (s, 3H).

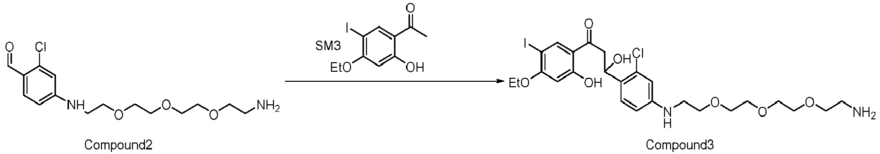

4.2 g of SM2 was added with 100mL THF and 20 mL methanol, 3.4g t-BuOK was added slowly at 0°C, the reaction was continued for 30 minutes at 0°C, 5.5 g Compound 2 was added continuously, the whole reaction was under Ar protection, and warmed to room temperature slowly to react for 24 h. Complete reaction of the raw materials and generation of the product were comfirmed by liquid quality monitor, added water to quench the reaction, extracted with ethyl acetate, dried with NaSO₄, and spin-dried. The crude product was directly charged to the next step 18g. MS: 639

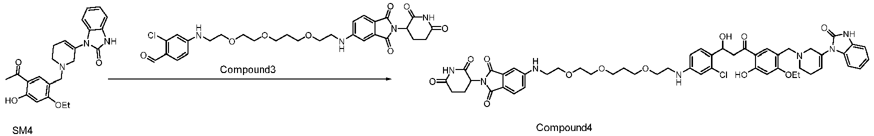

94 mg of SM4 was added with 5 ml DMSO and 1 ml THF, and 62 mg of potassium tert-butoxide was added under Ar protection at -10°C and stirried for 60min at 0°C. Compound 3 (135 mg, 1.0eq) was then added, allowed to warm to room temperature and stirred overnight. Monitored by LCMS untill no starting material remained. And slowly dripped the reaction solution into 1SM HCl aqueous solution for quenching, wherein the pH value of the system after quenching was between 6 and 7. Extracted by DCM:MeOH=10:1 mixed solvent, dried over anhydrous sodium sulfate, concentrated and spin dried. Prep-TLC purification with DCM:MeOH=10:1 gave a light yellow solid product 90 mg yield: 39.4% MS: [M-H] 1008.

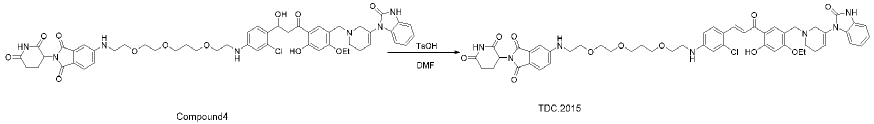

51 mg of compound 4 was added into 10ml DMF, followed by 28.5mg of anhydrous p-toluenesulfonic acid, heated to 110°C and stirried for 6 hours. Monitored by LCMS untill no starting material remained. Reverse column purification with ACN:H₂O(FA)=3:2 gave a white solid product 5mg (yield: 10.7%) MS: 990. HNMR ¹H NMR (400 MHz, CDCl₃). δ.9.51 (s, 1H), 7.97 (d, 1H), 7.82-7.74 (m, 2H), 7.72-7.65 (m, 1H), 7.58 (d, 1H), 7.53 (d, 1H), 7.45-7.38 (m, 1H), 7.36 (d, 1H), 7.27 (m, 1H), 7.16 (m, 1H), 6.94 (mm, 1H), 6.90 (d 1H), 6.69 (m, 1H), 6.56 (s, 1H), 5.50 (m, 1H), 5.24 (m, 1H), 5.09 (m, 1H), 4.66 (m, 1H), 4.08 (m, 2H), 3.83 (d, 2H), 3.67-3.62 (m, 6H) 3.59 (m, 2H), 3.52 (m, 4H), 3.47-3.38 (m, 6H), 2.94-2.87 (m, 2H), 2.90-2.81 (m, 1H), 2.64-2.49 (m, 2H), 2.15-2.03 (m, 2H), 1.85 (m, 2H), 1.43 m, 3H).

### Example 16 Synthesis of TDC2016

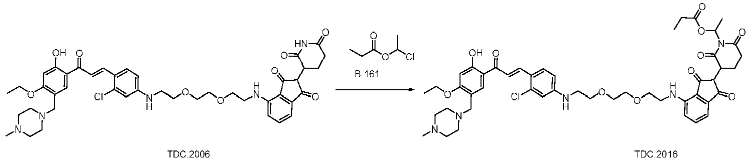

82 mg of TDC.2006 was dissolved into 10 ml DMF, followed by 15 mg of B-181, reacted at 80°C for 16 hours, and purified the mixture by column chromatography with EA n-hexane, 1~20%, to obtain the product 20 mg, yield: 25%, purity 92%, MS: ¹H NMR (400 MHz, CDCl₃) δ 8.00 (m, 1H), 7.97 (d, 1H), 7.80 (m, 1H), 7.58 (d, 1H), 7.53 (d, 1H), 7.44 (m, 1H), 7.37 m, 1H), 7.06-6.96 (m, 2H), 6.90 (ddd, 1H), 6.69 (m, 1H), 6.56 (s, 1H), 4.76 (d, 1H), 4.63 (m, 1H), 4.08 (m, 2H), 3.80-3.70 (m, 3H), 3.65 (s, 4H), 3.64 (m, 4H), 3.50 (m, 2H), 3.42 (m, 2H) 2.67-2.54 (m, 2H), 2.54-2.47 (m, 4H), 2.47-2.41 (m, 4H), 2.34 (m, 2H), 2.25 (m, 1H), 2.11 (m, 1H), 1.58 (dd, 3H), 1.43 (m, 3H), 1.13 (m, 3H).

### Example 17 Synthesis of TDC2017

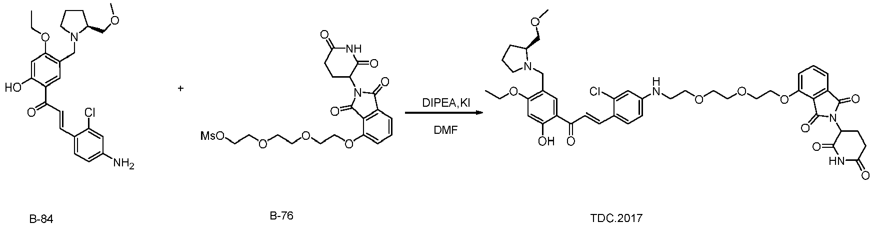

0.726mmol of B-84 was added into 3ml of DMF, followed by 1055mg of B-76 and 938mg of DIPEA, raised to 50°C and stirred, monitored by LC-MS until no B-84 remained. The system was separated and purified by Per-TLC (DCM:MeOH=20:1) to obtain a crude product, which was separated and purified by semi-preparative reverse phase separation and then by Per-TLC (DCM:MeOH=20:1) to obtain 24mg, purity: 85.07%, MS: 833. ¹H NMR (400 MHz, CDCl₃) δ 9.51 (s, 1H), 7.97 (d, 1H), 7.80 (m, 1H), 7.74 (m, 1H), 7.61-7.44 (m, 3H), 7.05 (m, 1H), 6.90 (d, 1H), 6.69 (m, 1H), 6.56 (s, 1H), 5.41 (mm, 1H), 4.63 (m, 1H), 4.18 (m, 2H), 4.08 (mm, 2H), 3.85-3.75 (m, 3H), 3.70 (m, 1H), 3.67 (dd, 2H), 3.67-3.60 (m, 4H), 3.42 (m, 2H), 3.39-3.27 (m, 2H), 3.31 (s, 3H), 2.92-2.79 (m, 3H), 2.73-2.64 (m, 1H), 2.64-2.49 (m, 2H), 2.15-2.03 (m, 1H), 1.82-1.73 (m, 1H), 1.77-1.69 (m, 2H), 1.73-1.62 (m, 1H), 1.43 (m, 3H).

### Example 18 Synthesis of TDC2018

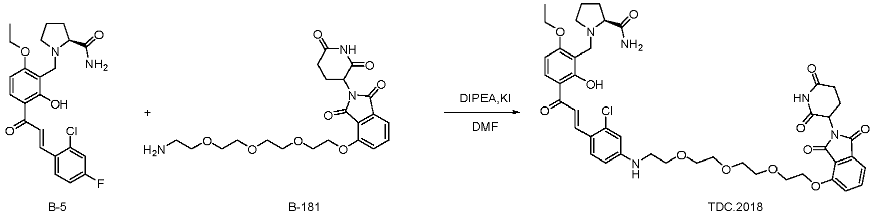

44.6mg of B-5 was added with 100mL of THF and 20 mL of methanol, slowly added 34 mg of t-BuOK at 0°C, continuously reacted for 30 minutes at 60°C, continuously added 80mg of B-181, the whole reaction was under the protection of Ar gas, and slowly raised to room temperature and reacted for 24 hours. Complete reaction of the raw materials and generation of the product were comfirmed by liquid quality monitor, added water to quench the reaction, extracted by ethyl acetate, dried with NaSO₄, and spin-dried. The crude product was directly charged to the next step 18 mg. MS: 876. ¹H NMR (400 MHz, CDCl₃) δ 9.51 (s, 1H), 7.95 (d, 1H), 7.78-7.69 (m, 2H), 7.61-7.44 (m, 3H), 7.05 (m, 1H), 6.90 (d, 1H), 6.72-6.64 (m, 2H), 6.54 (s, 2H), 5.41 (m, 1H), 4.63 (m, 1H), 4.18 (m, 2H), 4.08 (m, 2H), 3.92 (d, 1H), 3.82-3.73 (m, 3H), 3.73-3.66 (m, 2H), 3.70-3.60 (m, 8H), 3.46-3.38 (m, 3H), 2.90 (m, 2H), 2.91-2.81 (m, 1H), 2.64-2.49 (m, 2H), 2.15-2.04 (m, 1H), 2.04-1.87 (m, 2H), 1.87-1.74 (m, 2H), 1.43 (m, 3H).

### Example 19 Synthesis of TDC2019

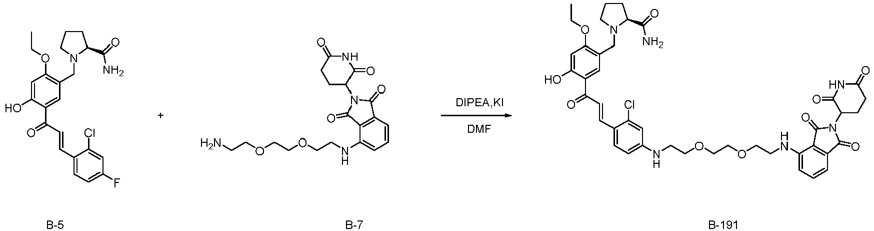

95mg of B-5 was added to 2ml of ultra dry DMF, followed by 269mg of B-7 and 245 mg of DIPEA, the temperature was raised to 60°C and stirred, and monitored by LC-MS until no B-5 remained. The reaction was applied in a thin layer and purified by Per-TLC (DCM:MeOH=20:1) to obtain 30mg of the product (purity: 95.4%), MS: 876. ¹HNMR (400 MHz, DMSO-d6) δ 11.1047 (s, 1H), 9.0103 (m, 1H), 8.3235 (br s, 1H), 8.1298-8.0989 (d, 2H), 7.9523-7.8647 (t, 1H), 7.8320-7.7925 (t, 1H), 7.5516-7.5302 (d, 1H), 7.4647-7.4466 (d, 1H), 6.9990 (s, 1H), 6.7225-6.6998 (d, 1H), 5.1063-5.0741 (d, 1H), 4.3647-4.3417 (t, 2H), 4.2337-4.1998 (t, 2H), 3.8350-3.8121 (t, 2H), 3.7441 (s, 1H), 3.6772-3.6358 (t, h) 3.5610-3.5372 (m, 2H), 3.3262 (s, 2H), 2.9270-2.8351 (q, 2H), 2.7759-2.7316 (d, 2H), 2.5601-2.5255 (t, 4H), 2.0461-1.9713 (m, 2H), 1.6777 (br s, 2H), 1.3899-1.3553 (t, 2H).

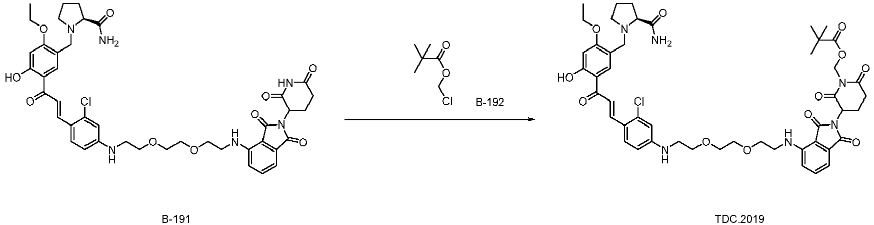

83 mg of B-191 was dissolved into 10 ml DMF, followed by 25 mg of B-181, reacted at 80°C for 16 hours, purified by column chromatography with EA n-hexane, 1~20%, to obtain the product 9 mg with a yield of 12% purity of 92%. ¹H NMR (400 MHz, CDCl₃) δ 7.97 (d, 1H), 7.79 (mm, 1H), 7.71 (m, 1H), 7.61-7.49 (m, 3H), 7.45 (m, 1H), 7.09 (m, 1H), 6.90 (d, 1H), 6.69 (m, 1H), 6.55 (d, 3H), 5.82 (m, 1H), 5.79 (s, 2H), 4.63 (m, 1H), 4.08 (m, 2H), 3.89 (m, 1H), 3.73 (m, 1H), 3.68-3.60 (m, 8H), 3.50 (m, 2H), 3.42 (m, 2H), 3.36 (m, 1H), 2.93-2.78 (m, 2H), 2.68-2.55 (m, 2H), 2.29-2.11 (m, 2H), 2.04-1.87 (m, 2H), 1.87-1.75 (m, 2H), 1.43 (m, 3H), 1.19 (s, 7H).

### Example 20 Synthesis of TDC2020

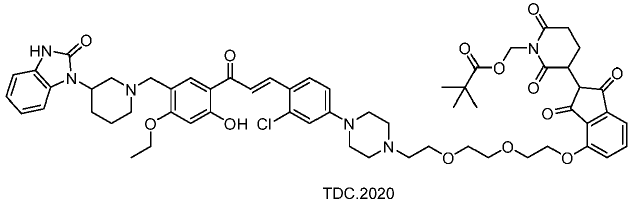

Method as before, MS: 1118.68 NMR: ¹H NMR (400 MHz, CDCl₃) δ 7.97 (dd, 1H), 7.87 (m, 1H), 7.80 (m, 1H), 7.58-7.50 (m, 2H), 7.50-7.42 (m, 1H), 7.46-7.39 (m, 1H), 7.39-7.32 (m, 1H), 7.26 (dm, 1H), 7.21-7.08 (m, 2H), 6.97 (dd, 1H), 6.80 (m, 1H), 6.56 (s, 1H), 5.84 (s, 2H), 4.56-4.45 (m, 2H), 4.19 (mm, 2H), 4.12-4.00 (m, dm 3H), 3.85 (dm, 1H), 3.82-3.72 (m, 3H), 3.71-3.61 (m, 4H), 3.55 (m, 2H), 3.30-3.23 (m, 4H), 3.13 (m, 1H), 3.02 (m, 1H), 2.81-2.71 (m, 4H), 2.75-2.62 (m, 4H), 2.65-2.54 (m, 2H), 2.24 (m, 1H), 2.17-2.04 (m, 1H), 1.89 (mm, 2H), 1.77-1.62 (m, 2H), 1.43 (mm, 3H), 1.19 (s, 7H).

### Pharmacological Activity test

The biological activity of each compound of the present invention is determined by the following method.

Reagent: complete DMEM medium, Gibco products. Fetal bovine serum, manufactured by ThermoFisher. Trypsin, manufactured by ThermoFisher. The SHP-2 antibody is produced by CST.

Cell line, MV 411, the blast cells of patients with bi-phenotypic B-myelomonocytic leukemia, was established by Rovera.

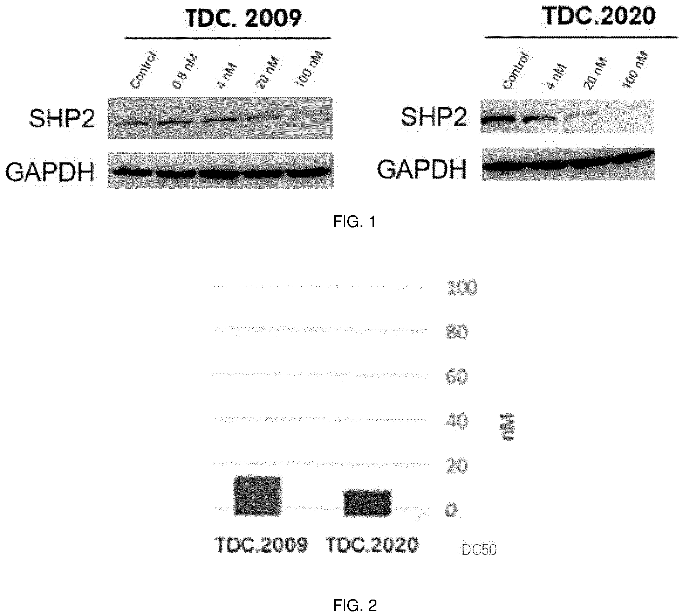

Measuring method: took the cells growing in the logarithmic phase, collected and suspended the cells in DMEM containing 10% fetal calf serum, gently blowed and beated the cells into single cell suspension by using a pipette, and counted the living cells under a microscope. 9 ml of the cultured cell suspension is inoculated into a 10 cm culture dish, the final concentration of the cells is 2×10⁵/ml, after pre-culturing for 24 hours under the culture condition of 37°C and 5% CO₂, DMSO solutions (the final concentration of the DMSO does not exceed 0.5%) of the compounds (TDC.2009 and TDC.2020) with different concentrations were added, and the negative control group was added with the same volume of DMSO. Then cultured for 24 hours. All cells were collected and washed with PBS, lysed with RIPA lysate and collected into a 1.7 ml tube. 95% metal bath for 5 minutes to fully denature the protein. 100V constant voltage electrophoresis after Western condensation and loading, stopped when bromophenol blue band reached the end point. The cellulose acetate membrane is transferred by a wet method, 45 minutes at a constant flow of 300-500 mA, 5% BSA is used for blocking for 1-2 hours at room temperature. Primary antibody of 1:500-1:8000 were used for incubation at 25°C for 40 minutes and then washed three times. Secondary antibody was used for incubation for 15-40 min at room temperature. The proteins were detected using ultrasensitive ECL luminophores such as Enlight, using an X-Ray film automatic developing machine. See the statistical tables below, and figures 1 and 2 for test results.

Effective concentration of the compound of the invention in the cell line for inhibiting SHP2

| | MV411 DC50 |
|---|---|
| TDC.2001 | 128 nM |
| TDC.2002 | 336 nM |
| TDC.2003 | 52 nM |
| TDC.2004 | 120 nM |
| TDC.2005 | 321 nM |
| TDC.2006 | 445 nM |
| TDC.2007 | 125 nM |
| TDC.2008 | 215 nM |
| TDC.2009 | 16 nM |
| TDC.2010 | 81 nM |
| TDC.2011 | 138 nM |
| TDC.2012 | 10 nM |
| TDC.2013 | 203 nM |
| TDC.2014 | 102 nM |
| TDC.2015 | 97 nM |
| TDC.2016 | 356 nM |
| TDC.2017 | 183 nM |
| TDC.2018 | 142 nM |
| TDC. 2020 | 13 nM |

The result shows that the compound of the invention has better in-vivo and in-vitro anti-tumor activity. The compounds of the invention are less toxic to normal cells.

The present invention is illustrated in detail by the examples given above, but the present invention is not limited to the details given above, which does not mean that the present invention must rely on the above detailed methods to be implemented. It should be understood by those skilled in the art that any modifications of the present invention, equivalent substitutions of the raw materials of the product of the present invention, and the addition of auxiliary components, selection of specific modes, etc., are within the scope and disclosure of the present invention.

## Claims

1. A compound of formula 1 or a pharmaceutically acceptable salt, ester, optical isomer, stereoisomer, polymorph, solvate, N-oxide, isotopically labeled compound, metabolite, chelate, complex, clathrate, or prodrug thereof, 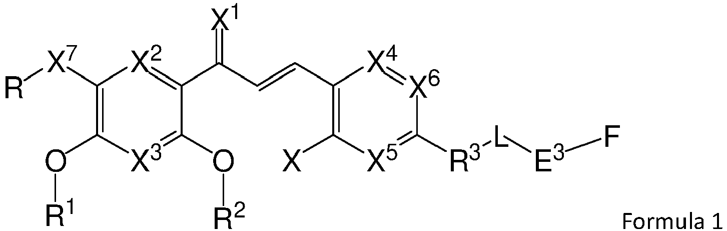
In formula 1,
X is halogen, C1-6 alkyl, C2-6 alkenyl, C2-6 alkynyl, saturated or partially unsaturated C3-10 cyclic hydrocarbyl, preferably halogen, methyl, ethyl, isopropyl, cyclopropyl, more preferably halogen,
X¹ is NR⁴, O or S, preferably O or S, more preferably O,
X⁷ is one selected from a chemical bond, C1-6 alkylene, C2-6 alkenylene, C2-6 alkynylene, saturated or partially unsaturated C3-10 cyclic hydrocarbylene, -O-, -CO-, -C(=O)O-, -CONH-, -NHCO-, -NHCONH-, -NH-, -S-, sulfinyl and sulfonyl, preferably C1-6 alkylene, and more preferably methylene, ethylene, propylene, -O-,
X², X³, X⁴, X⁵ and X⁶ are each independently CR⁴ or N, preferably CR⁶,
R¹ is H, C1-6 alkyl, C2-6 alkenyl, C2-6 alkynyl, saturated or partially unsaturated C3-10 cyclic hydrocarbyl, preferably C1-6 alkyl, more preferably one selected from methyl, ethyl, isopropyl and cyclopropyl,
R² is H, C1-6 alkyl, C2-6 alkenyl, C2-6 alkynyl, saturated or partially unsaturated C3-10 cyclic hydrocarbyl, preferably H, C1-6 alkyl, more preferably one selected from H, methyl and ethyl, particularly preferably H,
R³ is CR⁴R⁵, NR⁴, 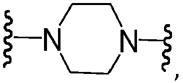 O or S, preferably NR⁴, 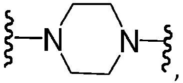 O or S, more preferably NR⁴, 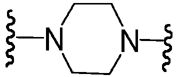 or O,
R⁴ and R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R⁶ is H, halogen, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl, or C6-12 aralkyl,
R is H, halogen, NH₂, OH, SH, amino, substituted or unsubstituted C2-10 aliphatic hydrocarbon group, substituted or unsubstituted saturated or partially unsaturated 3-10 membered heterocyclyl, substituted or unsubstituted C6-10 aryl, or substituted or unsubstituted 5-14 membered heteroaryl;
R is preferably halogen, OH, SH, haloalkyl, amino, saturated C3-10 cyclic hydrocarbyl, saturated or partially unsaturated 3-10 membered heterocyclyl, C1-6 alkyl substituted amino, 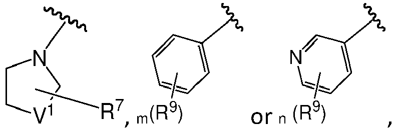
Wherein V¹ is one selected from C(R⁸)₂, C(R⁸)₂-C(R⁸)₂, CR⁸=CR⁸, C=O, C(=O)C(R⁸)₂, C(R⁸)₂C(=O), C(=O)O, OC(=O), C(=O)NR, N=CR⁸, CR⁸=N, NR⁸-C(R⁸)₂ or C(R⁸)₂ -NR⁸;
R⁷ is one selected from H, halogen, cyano, nitro, hydroxylmethyl, hydroxyl, amide, C1-C6 alkyl, C1-C6 alkoxy, C1-C6 alkoxy-C1-C6 alkylene, or 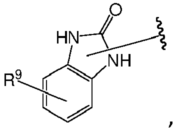
R⁹ are each independently selected from the group consisting of halogen, hydroxyl, oxo, amino, cyano, nitro, -Si(R⁸)₃, C1-6 alkyl, C1-6 alkoxy, saturated or partially unsaturated C3-6 cyclic hydrocarbyl, saturated or partially unsaturated 3-10 membered heterocyclyl, C6-10 aryl, 5-14 membered heteroaryl, C6-12 aralkyl, -C(=O)R⁸, -OC(=O)R⁸, -C(=O)OR⁸, - OR⁸, -SR⁸, -S(=O)R⁸, -S(=O)₂R⁸, -S(=O)₂N(R⁸)₂, -N(R⁸)₂, -C(=O)N(R⁸)₂, -NR⁸-C(=O)R⁸, -NR⁸-C(=O)OR⁸, -NR⁸-S(=O)₂-R⁸, -NR⁸-C(=O)-N(R⁸)₂, -C1-6 alkylene -N(R⁸)₂, -C1-6 alkylene-OR⁸, -C1-6 alkenylene-OR⁸ and -O-C1-6 alkylene-N(R⁸)₂; m is an integer of 0 to 5, n is an integer of 0 to 4,
R⁸ is H, C1-C6 alkyl, saturated or partially unsaturated C₃₋₆ cyclic hydrocarbyl, saturated or partially unsaturated 3-10 membered heterocyclyl, or C6-10 aryl, the expression of the ring structure with "-" represents the connecting site at any position on the ring structure that can form a bond;
L is a linking group which represents a linear or branched C3-C29 alkylene chain, wherein said linear or branched C3-C29 alkylene chain is optionally interrupted one or more times by one or more divalent groups selected from-O-, -CO-, -C(=O)O-, -CONH-, -NHCO-, -NHCONH-, -NH-, -NR⁸-, -C(R⁸)₂-, -S-, sulfinyl, sulfonyl, sulfinyloxy, sulfonyloxy, - aminosulfonylamino - , alkynylene, alkenylene, cyclic hydrocarbylene, 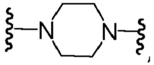 or any combination thereof,
E³ is the following group, 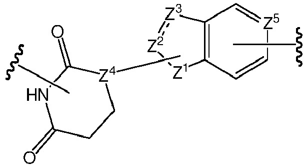
-Represents a single or double bond, preferably a single bond;
Z¹ is O, S, NH, CH₂ or C=O, preferably CH₂ or C=O;
When -̅-̅-̅-̅-̅ is a double bond, Z² is N or CH, and Z³ is N or CH;
When -̅-̅-̅-̅-̅ is a single bond, Z² is O, S, NH, CH₂ or C=O, preferably NH; z³ is O, S, NH, CH₂ or C=O, preferably CH₂ or C=O;
Z⁴ is N or CH, and Z⁴ is connected with any connectable position of Z¹, Z² and Z³;
Z⁵ is N or CH, preferably N;
E³ is more preferably 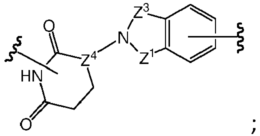
F is H or 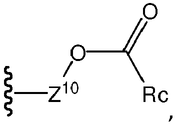
Z¹⁰ is selected from a chemical bond, C1-6 alkylene, C2-6 alkenylene, C2-6 alkynylene, saturated or partially unsaturated C3-10 cyclic hydrocarbylene, preferably a bond, C1-6 alkylene;
R_{c} is halogen, cyano, nitro, hydroxyl, amido, C1-C6 alkyl, C1-C6 alkoxy, substituted C1-C6 alkyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl, preferably C1-C6 alkyl, saturated or partially unsaturated C3-10 cyclic hydrocarbyl, C6-10 aryl, C6-12 aralkyl,
In the above expression, 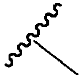 denotes the position of the linkage, and the expression of the ring straucture with "-" represents the connecting site at any position on the ring structure that can form a bond.

2. The compound of claim 1, or a pharmaceutically acceptable salt, ester, optical isomer, stereoisomer, polymorph, solvate, N-oxide, isotopically labeled compound, metabolite, chelate, complex, clathrate, or prodrug thereof, having a structure of formula 2 below, 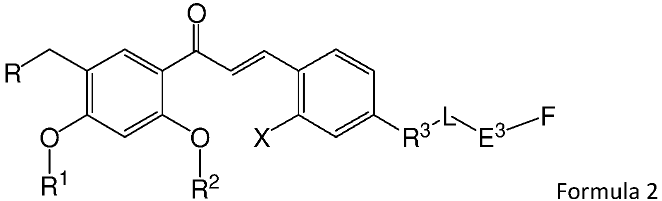
In formula 2, X is halogen, C1-6 alkyl, saturated or partially unsaturated C3-10 cyclic hydrocarbyl, preferably halogen,
In formula 2, R¹ is H, C1-6 alkyl, C2-6 alkenyl, C2-6 alkynyl, saturated or partially unsaturated C3-10 cyclic hydrocarbyl, preferably C1-6 alkyl, further preferably ethyl,
In formula 2, R² is H, C1-6 alkyl, C2-6 alkenyl, C2-6 alkynyl, saturated or partially unsaturated C3-10 cyclic hydrocarbyl, preferably H,
In formula 2, R³ is NR⁴, 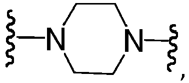 O or S, preferably NR⁴, 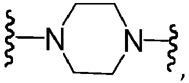 O or S, further preferably NR⁴, 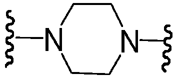 or O, in formula 2, R⁴ are each independently H, C1-6 alkyl, R⁴ is preferably H,
In formula 2, R, L, E³ and F have the same meanings as in claim 1, and in the above expression, 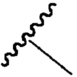 represents a connecting site.

3. The compound of claim 1, or a pharmaceutically acceptable salt, ester, optical isomer, stereoisomer, polymorph, solvate, N-oxide, isotopically labeled compound, metabolite, chelate, complex, clathrate, or prodrug thereof, having a structure represented by formula 3 below, 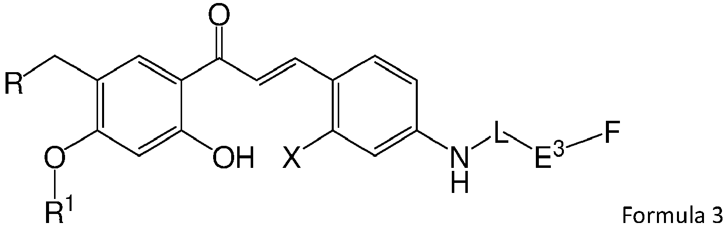
In formula 3, X is halogen, C1-6 alkyl, saturated or partially unsaturated C3-10 cyclic hydrocarbyl, preferably halogen, R¹ is H, C1-6 alkyl, C2-6 alkenyl, C2-6 alkynyl, saturated or partially unsaturated C3-10 cyclic hydrocarbyl, preferably C1-6 alkyl, further preferably ethyl,
In formula 3, R, L, E³ and F have the same meanings as in claim 1.

4. The compound of claim 1, or a pharmaceutically acceptable salt, ester, optical isomer, stereoisomer, polymorph, solvate, N-oxide, isotopically labeled compound, metabolite, chelate, complex, clathrate, or prodrug thereof, having a structure represented by formula 4 below, 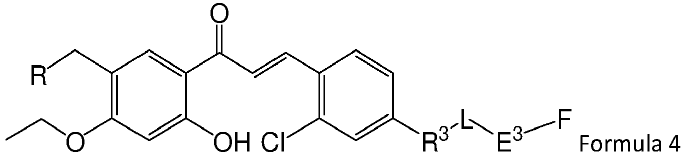
In formula 4, R³ is CR⁴R⁵, NH, 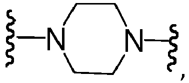 O or S, preferably NR⁴, 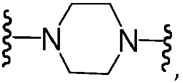 O or S, more preferably NH, 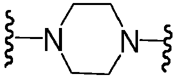 or O, R⁴, R⁵ are each independently H, C1-6 alkyl, C2-6 alkenyl, C2-6 alkynyl, saturated or partially unsaturated C3-10 cyclic hydrocarbyl, saturated or partially unsaturated 3-10 membered heterocyclyl, C6-10 aryl, 5-14 membered heteroaryl or C6-12 aralkyl,
In formula 4, R, L, E³ and F have the same meanings as in claim 1, and in the above expression, 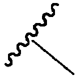 represents a connecting site.

5. The compound of any one of claims 1-4, or a pharmaceutically acceptable salt, ester, optical isomer, stereoisomer, polymorph, solvate, N-oxide, isotopically-labeled compound, metabolite, chelate, complex, clathrate, or prodrug thereof,
E³ is one selected from the following groups, 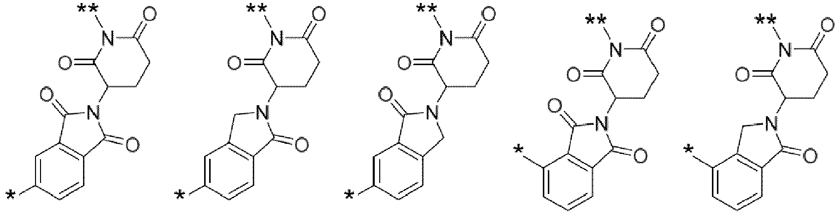 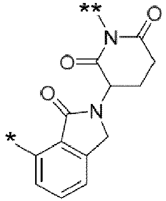
The above groups are linked to L via one of the two positions labeled with *- or **- and the other position is linked to F,
F is H or one of the following groups,

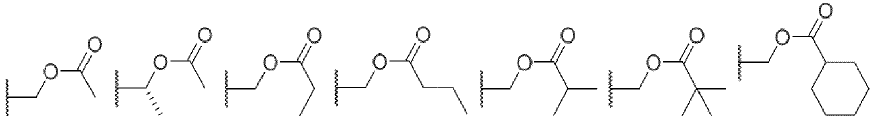

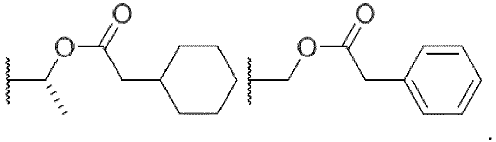

6. The compound of any one of claims 1-5, or a pharmaceutically acceptable salt, ester, optical isomer, stereoisomer, polymorph, solvate, N-oxide, isotopically-labeled compound, metabolite, chelate, complex, clathrate, or prodrug thereof,
R is halogen, OH or 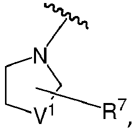 wherein V¹ is one selected from CH₂, CH₂-CH₂, CH = CH, NH-CH₂ or CH₂-NH; R⁷ is one selected from H, C1-C6 alkyl, C1-C6 alkoxy substituted C1-C6 alkylene or 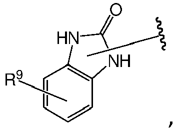 R⁹ is as defined in claim 1. 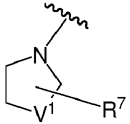 is preferably one selected from 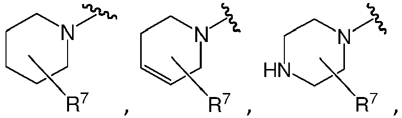 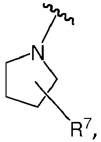 wherein R⁷ is further preferably one selected from H, methyl, ethyl, 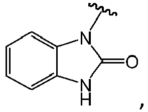 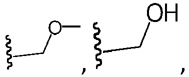 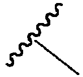 represents a connecting site.

7. The compound of claim 1, or a pharmaceutically acceptable salt, ester, optical isomer, stereoisomer, polymorph, solvate, N-oxide, isotopically labeled compound, metabolite, chelate, complex, clathrate, or prodrug thereof, wherein R is one of the following gorups, 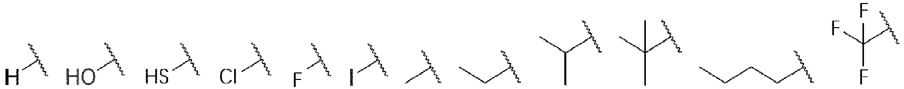 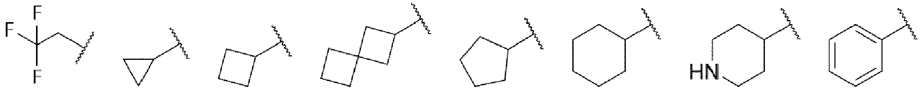 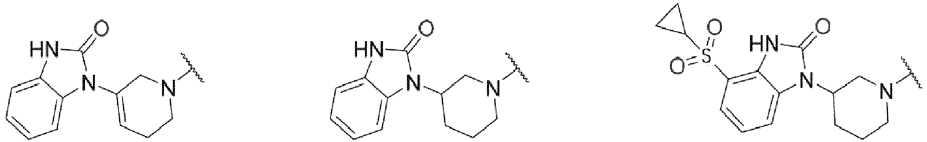 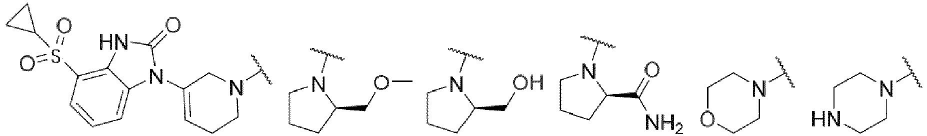 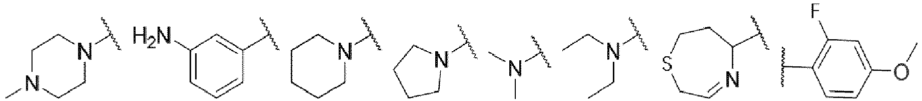 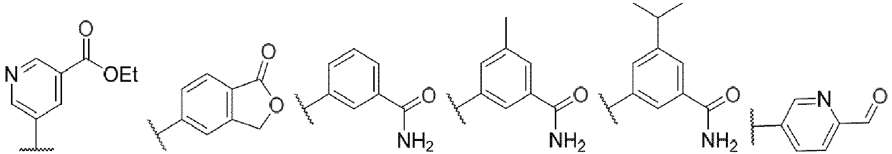 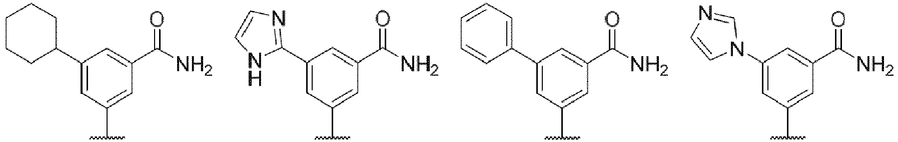 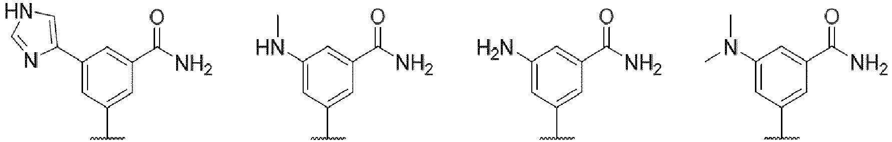 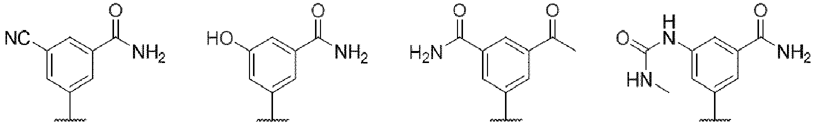 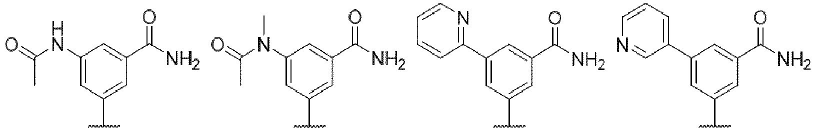 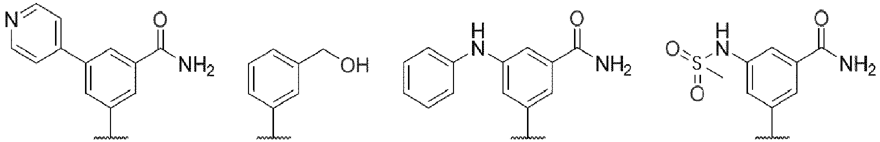 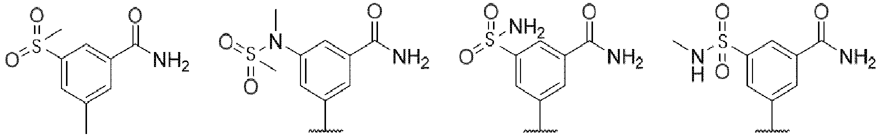 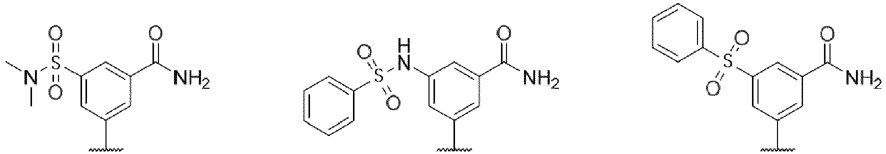 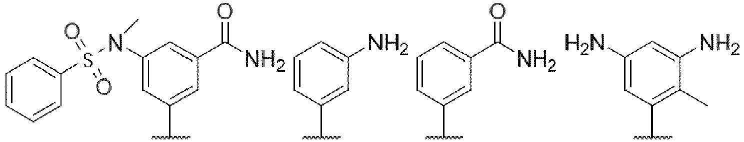 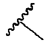 represents a connecting site.

8. The compound of any one of claims 1-5, or a pharmaceutically acceptable salt, ester, optical isomer, stereoisomer, polymorph, solvate, N-oxide, isotopically-labeled compound, metabolite, chelate, complex, clathrate, or prodrug thereof, wherein L is a divalent group represented by formula 5, 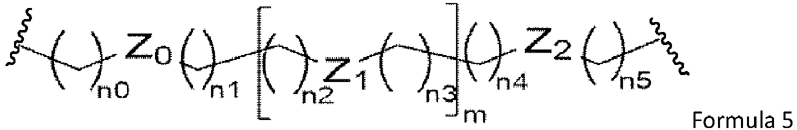
n₀ is 0 or 1,
m is an integer from 1 to 5, preferably 2 or 3; n₁ is an integer from 0 to 3, preferably 1; n₂ is an integer of 0 to 3, preferably 1; n₃ is an integer from 0 to 3, preferably 1; n₄ is an integer of 0 to 3, preferably 1; n₅ is an integer of 0 to 3, preferably 1.
Z₀ is -CH₂-, -NH-, -O-, -S-, 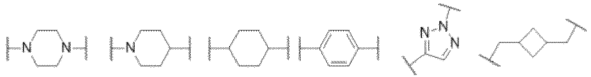 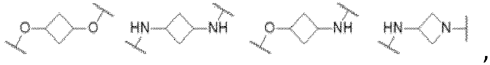 -CO- or -C(=O)O-; Z₁ is -CH₂-, -NH-, -O-, -S-, 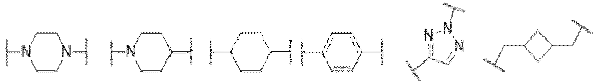 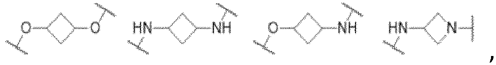 -CO- or -C(=O)O-; Z₂ is -CH₂-, -NH-, -O-, -S-, 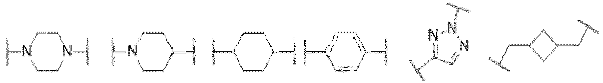 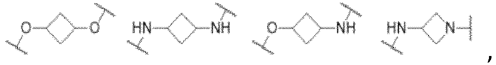 -CO- or -C(=O)O-; 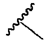 represents a connecting site, and the connecting directions of the two ends are arbitrarily changed.

9. The compound of claim 8, or a pharmaceutically acceptable salt, ester, optical isomer, stereoisomer, polymorph, solvate, N-oxide, isotopically labeled compound, metabolite, chelate, complex, clathrate, or prodrug thereof, wherein L is one of the following divalent groups, 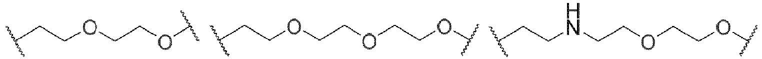 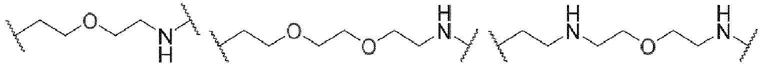 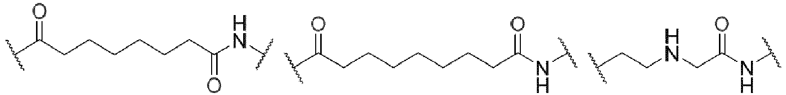 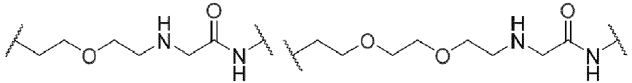 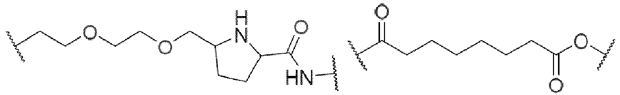 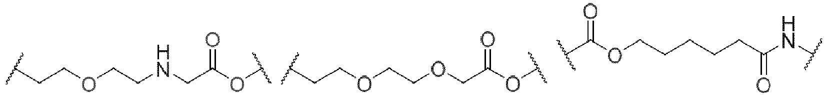 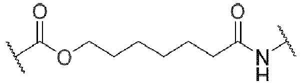 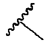 represnets connecting site, and the connecting directions of the two ends are arbitrarily changed.

10. The compound of claim 1, or a pharmaceutically acceptable salt, ester, optical isomer, stereoisomer, polymorph, solvate, N-oxide, isotopically labeled compound, metabolite, chelate, complex, clathrate, or prodrug thereof, wherein the compound has the structure below,

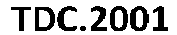

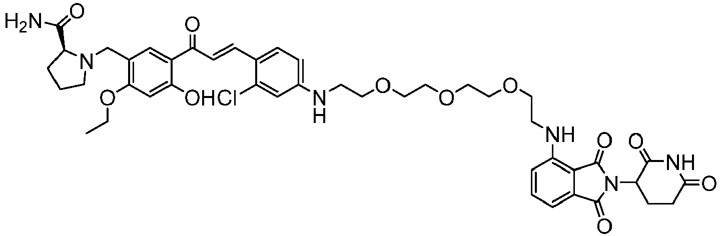

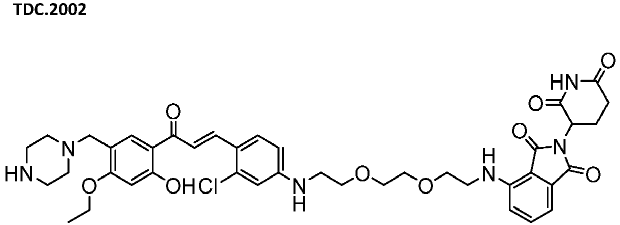

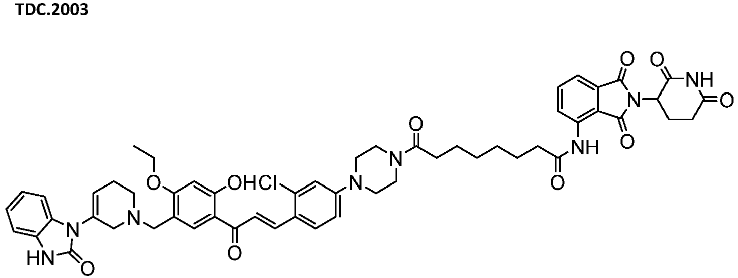

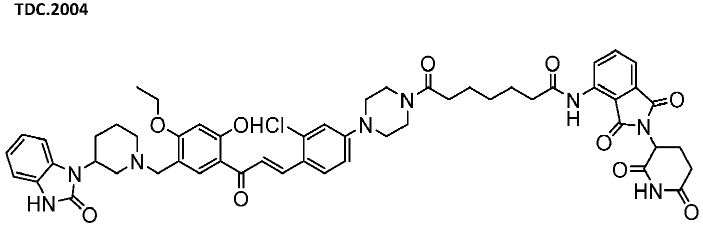

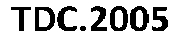

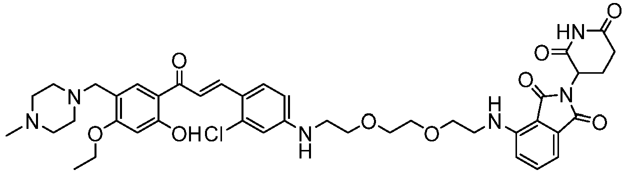

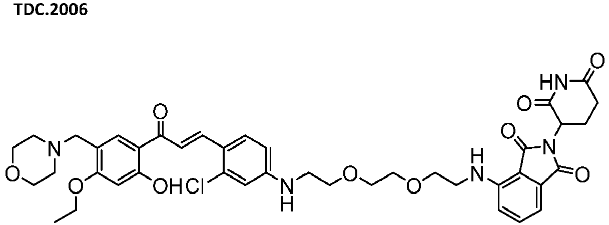

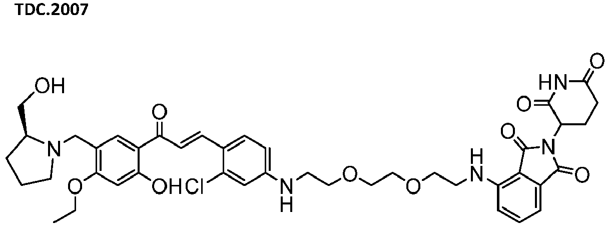

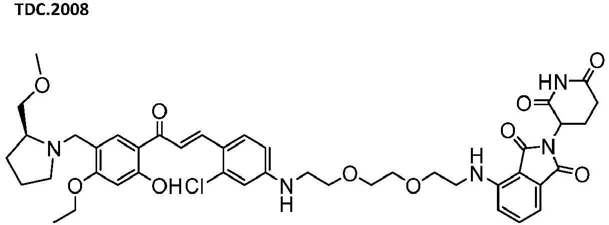

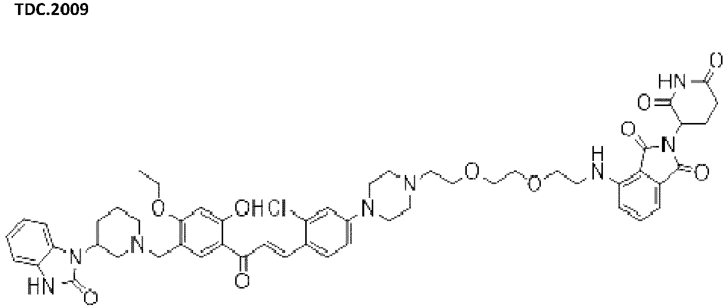

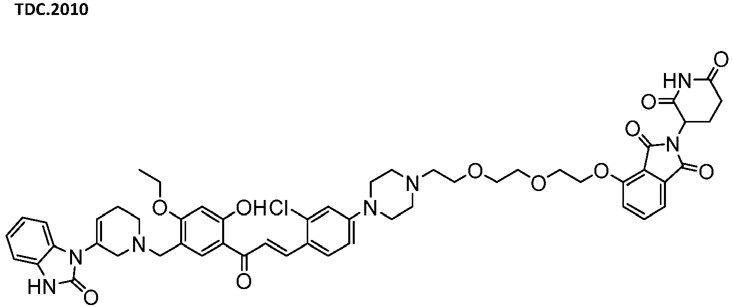

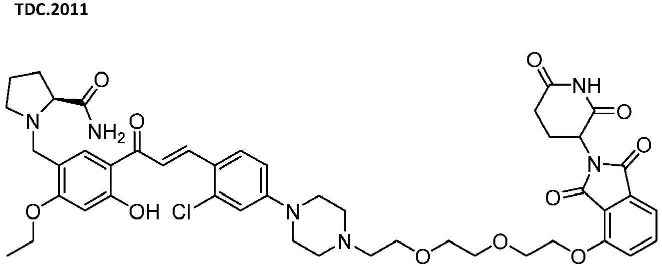

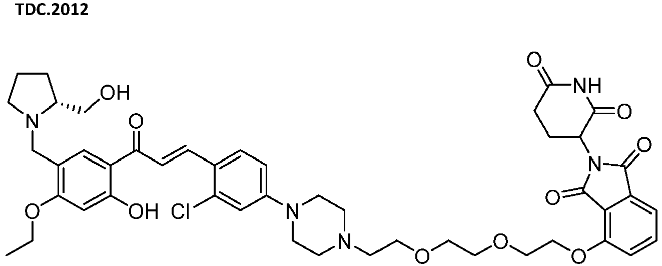

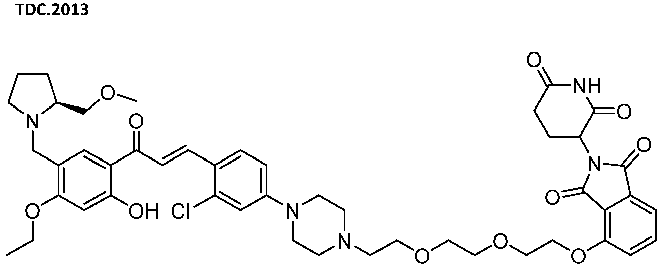

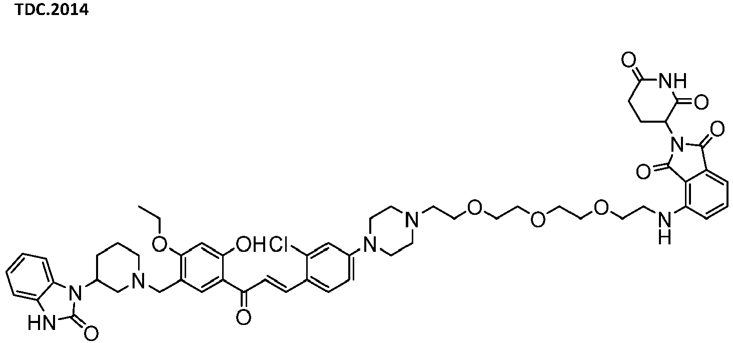

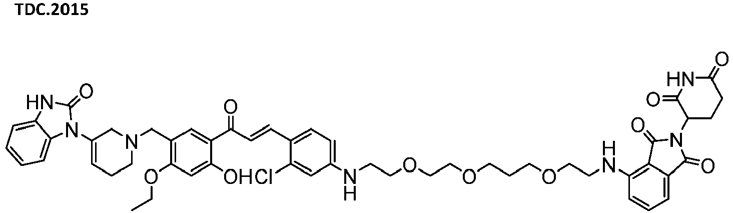

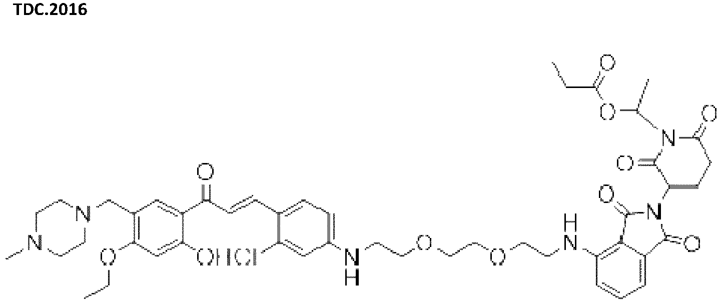

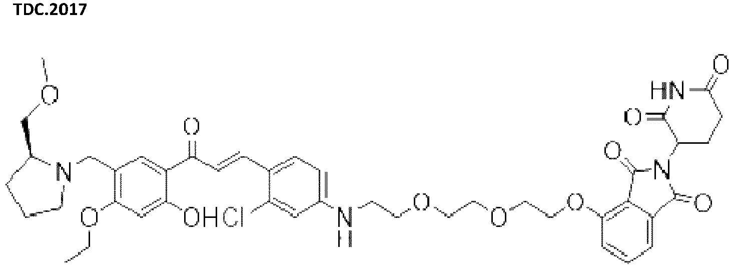

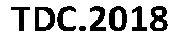

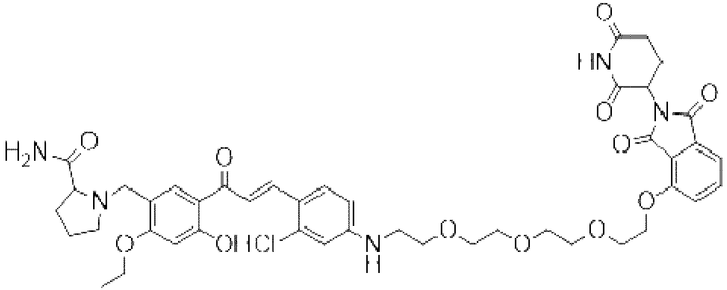

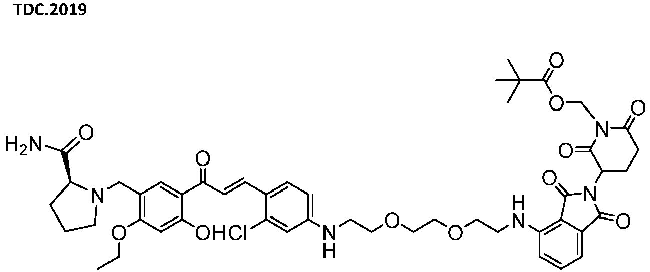

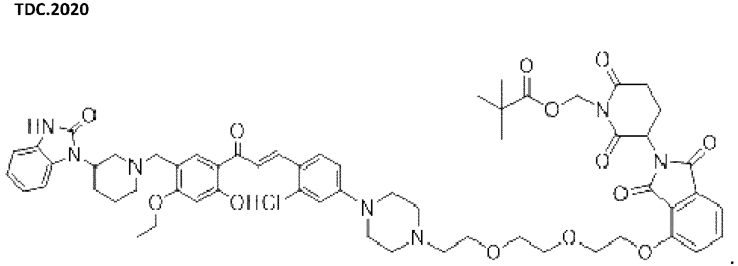

11. A pharmaceutical composition comprising a prophylactically or therapeutically effective amount of the compound according to any one of claims 1-10, or a pharmaceutically acceptable salt, ester, optical isomer, stereoisomer, polymorph, solvate, N-oxide, isotopically labeled compound, metabolite, chelate, complex, clathrate, or prodrug thereof, and a pharmaceutically acceptable carrier, said pharmaceutical composition is preferably in a solid, semi-solid, liquid, or gaseous formulation.

12. Use of the compound of any one of claims 1-10, or a pharmaceutically acceptable salt, ester, optical isomer, stereoisomer, polymorph, solvate, N-oxide, isotopically-labeled compound, metabolite, chelate, complex, clathrate, or prodrug thereof, or the pharmaceutical composition of claim 11 in the manufacture of a medicament for the treatment of a SHP2 phosphatase modulated disease.

13. The use according to claim 12, wherein the disease is a tumor, such as a solid tumor, a hematological tumor, a malignant tumor, a refractory tumor, a primary or metastatic recurrent tumor, and the like.

14. The use according to claim 12, wherein the disease is selected from primary or metastatic recurrent NSCLC, squamous carcinoma of the lung, adenocarcinoma of the lung, squamous carcinoma of the head and neck, gastric cancer, colorectal cancer, pancreatic cancer, and the like; also included are breast cancer, esophageal cancer, lung cancer, colon cancer, brain cancer, neuroblastoma, neuroblastoma, melanoma, squamous carcinoma of the head and neck, anaplastic large cell lymphoma and glioblastoma, hematological malignancies of cachexia including juvenile myelomonocytic leukemia, acute myelogenous leukemia, noonan syndrome, leopard syndrome, type II diabetes, and obesity.

15. A method of treatment of a SHP2 phosphatase modulated disease, comprising administering to a human in need of such treatment an effective amount of a compound of any one of claims 1-10 or a pharmaceutically acceptable salt, ester, optical isomer, stereoisomer, polymorph, solvate, N-oxide, isotopically labeled compound, metabolite, chelate, complex, clathrate, or prodrug thereof, or a pharmaceutical composition of claim 11.

16. The method of treatment according to claim 15, wherein the disease is a tumor, such as a solid tumor, a hematological tumor, a malignant tumor, a refractory tumor, a primary or metastatic recurrent tumor, and the like.

17. The method of treatment of claim 15, wherein the disease is selected from primary or metastatic recurrent NSCLC, squamous carcinoma of the lung, adenocarcinoma of the lung, squamous carcinoma of the head and neck, gastric cancer, colorectal cancer, pancreatic cancer, and the like; also included are breast cancer, esophageal cancer, lung cancer, colon cancer, brain cancer, neuroblastoma, neuroblastoma, melanoma, squamous carcinoma of the head and neck, anaplastic large cell lymphoma and glioblastoma, hematological malignancies of cachexia including juvenile myelomonocytic leukemia, acute myelogenous leukemia, noonan syndrome, leopard syndrome, type II diabetes, and obesity.
